(19) Europäisches Patentamt / European Patent Office / Office européen des brevets

(11) **EP 2 761 359 B1**

(12) **EUROPÄISCHE PATENTSCHRIFT**

(45) Veröffentlichungstag und Bekanntmachung des Hinweises auf die Patenterteilung:
**23.09.2020  Patentblatt 2020/39**

(21) Anmeldenummer: **12772226.2**

(22) Anmeldetag: **26.09.2012**

(51) Int Cl.:
**G02B 27/00** (2006.01)     **G02C 7/06** (2006.01)
**A61F 2/16** (2006.01)     **G02B 5/18** (2006.01)

(86) Internationale Anmeldenummer:
**PCT/EP2012/004026**

(87) Internationale Veröffentlichungsnummer:
**WO 2013/045079 (04.04.2013 Gazette 2013/14)**

(54) **LINSE MIT EINEM ERWEITERTEN FOKUSBEREICH**

LENS HAVING AN EXTENDED FOCAL RANGE

LENTILLE PRÉSENTANT UNE PLAGE DE DISTANCE FOCALE ÉTENDUE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **29.09.2011  DE 102011114752**

(43) Veröffentlichungstag der Anmeldung:
**06.08.2014  Patentblatt 2014/32**

(73) Patentinhaber: **Carl Zeiss AG**
**73447 Oberkochen (DE)**

(72) Erfinder: **DOBSCHAL, Hans-Jürgen**
**99510 Kleimromstedt (DE)**

(56) Entgegenhaltungen:
EP-A1- 0 622 653     WO-A1-2009/012789
WO-A2-2008/087486     GB-A- 2 370 653
US-A1- 2010 329 605     US-B1- 6 330 118

**Beschreibung**

Die Erfindung betrifft eine Linse, welche einen erweiterten Fokusbereich aufweist, wobei die Linse aus einem festen Material besteht, die optischen Flächen der Linse transparent sind und die Linse eine Brechkraftverteilung aufweist. Weiterhin betrifft die Erfindung ein Verfahren zur Herstellung der Linse sowie deren Verwendung zur Beeinflussung der Abbildung eines Bildes auf der Retina eines Auges sowie deren Verwendung in einem Objektiv mit einem erweiterten Fokusbereich.

Multifokale Linsen sollen gleichzeitig mehrere Forderungen erfüllen. Zunächst soll eine hinreichend gute Kontrastübertragungsfunktion in zwei oder mehr Fokusebenen gewährleistet werden. Weiterhin soll die Kontrastübertragungsfunktion unabhängig von der Größe der Pupille sein. Und schließlich soll die Linse leicht herstellbar sein, sie soll keine Absätze sowie Kanten und daher möglichst glatte Kurven aufweisen.

Derartige Linsen werden insbesondere bei der Korrektur von Sehfehlern mittels Brillengläsern oder als Intraokularlinsen (IOL-Linsen) verwendet.

Im Gegensatz zu den schon seit vielen Jahren eingeführten monofokalen IOL-Linsen sind multifokale Linsen bisher nur für den bifokalen Fall umgesetzt, da es erhebliche Probleme bereitet, die oben genannten Forderungen gleichzeitig zu erfüllen. Eine Variante basiert hierbei auf einem speziellen rotationssymmetrischen Ringsystem, wobei durch eine geschickte Abstimmung von Ringradien, Ringbreiten und Ringtiefen eine ausreichend gute Abbildung für zwei diskrete objektseitige Brennebenen erfolgt, beispielsweise bei 0 dpt und ca. 3 dpt Korrekturstärke.

Eine derartige bifokale Linse wird in der US 5 982 543 A beschrieben und verwendet ein rotationssymmetrisches fresnel-ähnliches Ringsystem.

In US 6120148A wird ein rotationssymmetrisches diffraktives Ringsystem beschrieben. Die bifokale Linse aus US 6 536 899 B1 verwendet ebenfalls ein Ringsystem, wobei jeder Ring aus zwei Subringen besteht, die jeweils die zwei gewünschten Brennweiten realisieren.

In etwas modifizierter Form sind hieraus auch Lösungen abgeleitet, bei denen eine einzige Linse einen erweiterten, kontinuierlichen Fokusbereich abdeckt. Derartige Linsen sind auch unter dem Begriff "Extended Depth of Focus-Linse" oder auch "EDoF-Linse" bekannt. In US 2006 176 572 A1 wird ein rotationssymmetrisches System aus Ringen verwendet, wobei die Einzelbrennweiten der Ringe innerhalb des gewünschten kontinuierlichen Brennweitenbereiches liegen. Der "Extended depth of Focus"-Effekt entsteht durch die Vermischung der verschieden Brennweiten.

Das System gemäß WO 2010 083 546 A2 besteht aus Sektoren ("Tortenstücken") mit azimutal ansteigender Brechkraft. Die Brechkraftverteilung weist hierbei diskrete Sprünge zwischen den Sektoren auf. In EP 0 622 653 A1 ist eine Linse mit einer spiralförmigen Struktur offenbart, die eine Vielzahl von Brechkräften bereitstellt.

In US 2010 002 310 A1 wird ein optisches Abbildungssystem für eine Kamera beschrieben, welches einen erweiterten Schärfentiefenbereich aufweist. Die erweiterte Schärfentiefe wird durch eine Kombination mehrere Linsen mit asphärischen Oberflächen erreicht.

In EP 0 622 653 A1 ist eine multifokale Kontaktlinse beschrieben, welche ein spiralförmiges Muster auf ihrer Oberfläche aufweist.

Nachteilig ist insbesondere bei Intraokularlinsen, dass bei der Verwendung von "normalen" sphärischen oder asphärischen Linsengrundformen auf Grund der relativ kurzen, durch die Augenlänge bedingten Brennweite eine starke Radienkrümmung erforderlich ist. Dadurch entstehen eine große Linsendicke, ein relativ großes Linsenvolumen mit einem entsprechend großem Gewicht. Da Intraokularlinsen aus organischen Polymeren gefertigt werden, ist die Brechzahl meist relativ niedrig, was eine starke Radienkrümmung und damit auch eine relativ dicke Linsenform zu Folge hat.

Die Aufgabe der Erfindung besteht darin, eine neue Linse mit einem erweiterten Fokusbereich zu schaffen. Die neue Linse soll einzeln, insbesondere als Intraokularlinse oder in Verbindung mit anderen optischen Komponenten optische Systeme liefern, welche bei einer hinreichend guten Abbildungsqualität einen großen Schärfentiefenbereich liefern. Die neue Linse soll kostengünstig herstellbar sein.

Insbesondere soll die neue Linse bei einer Verwendung als Intraokularlinse bei einer vorgegebenen Brechkraft eine reduzierte Linsendicke aufweisen.

Die Aufgabe der Erfindung wird erfindungsgemäß für die neue Linse mit den kennzeichnenden Merkmalen des Anspruchs 1 gelöst. Die Aufgabe der Erfindung wird erfindungsgemäß bei dem Verfahren zur Herstellung der Linse mit den Merkmalen des Anspruchs 12 gelöst.

Die Merkmale in den jeweiligen Unteransprüchen sind vorteilhafte Ausgestaltungen der Merkmale in den unabhängigen Ansprüchen.

Die Aufgabe der Erfindung wird erfindungsgemäß bei einem Objektiv dadurch gelöst, dass eine Linse mit den kennzeichnenden Merkmalen des Anspruchs 17 im Strahlengang des Objektivs angeordnet ist.

Die Linse mit einem erweiterten Fokusbereich besteht aus einem festen, transparenten Material und hat zwei gefertigte optische Flächen. Erfindungsgemäß hat die Linse eine Brechkraftverteilung $F_{Ges}$, welche bezogen auf eine zur optischen Achse senkrecht stehende Ebene eine Funktion der radialen Höhe r und des Azimutwinkels der Apertur phi ist und sich zwischen einem Grundwert der Brechkraft $F_{Linse}$ ungleich Null und einem Maximalwert $F_{Spirale\ max}$ ändert.

Somit ergibt sich die Brechkraftverteilung zu $F_{Ges}(r, phi) = F_{Linse} + F_{Spirale}(r, phi)$, mit dem spiralförmigen Brechkraftanteil

$$F_{Spirale}(r, phi) = F_{Spirale\ max}(r, phi) * w(phi)$$

wobei $F_{Spirale\ max}(r,phi)$ nichtlinear radienabhängig ist und $w(phi)$ ein Faktor für den Brechkraftanteil mit einem spiralförmigen Verlauf ist.

Ein wesentlicher weiterer Aspekt der Erfindung ist, dass ein Wert der Brechkraft der Linse $F_{Linse}$ aufgeteilt wird, in einen Anteil der Brechkraft eines refraktiven Basissystems der Linse $F_{Basis}$ und einen strukturförmigen Anteil der Brechkraft $F_{Struktur}$, so dass gilt $F_{Linse} = F_{Basis} + F_{Struktur}$. Dabei ist $F_{Basis}$ eine Basisbrechkraft einer Linse, welche durch Linsenradien oder Freiformflächenpolynome und Linsendicke sowie Brechzahl des Linsenmaterials bestimmt ist, und $F_{Struktur}$ ist eine Brechkraft, die nicht durch Linsenradien oder Freiformflächenpolynome und Linsendicke sowie Brechzahl des Linsenmaterials beschrieben ist. In dieser Schrift wird daher der Begriff "Struktur" und das Formelzeichen $F_{Struktur}$ als Brechkraft einer Struktur definiert, die in einem ersten Fall als Höhenprofil $Z_{Fresnel}$ einer Fresnelschen Stufenlinse vorliegt, die in einem zweiten Fall als Phasenprofil $Phase_{Struktur}$ eines Diffraktiven Optischen Elementes (DOE) vorliegt oder in einem dritten Fall als Brechungsindexgradient $\Delta n_{Struktur}$ einer Gradienten-Index-Linse (GRIN-Linse) vorliegt. Die Brechkraftverteilung der erfindungsgemäßen Linse ergibt sich für den rotationssymmetrischen Fall somit zu

$$F_{Ges}(r, phi) = F_{Basis}(r) + F_{Struktur}(r) + F_{Spirale\ max}(r, phi) * w(phi).$$

Jedoch können sowohl die Basisbrechkraft der Linse $F_{Basis}$ als auch die Brechkraft der Struktur $F_{Struktur}$ den Brechkraftverlauf einer Freiformfläche aufweisen, wobei dann die entsprechenden Polynome in den Formeln einzusetzen sind.

Die Erfindung betrifft daher eine spezielle, neue Linsenform, mit der es möglich ist, einen vorgegebenen Brennweitenbereich simultan abzudecken, das heißt, über einen ausgedehnten Fokalbereich eine hinreichend gute Bildqualität zu generieren. Durch die Aufteilung des Grundwertes der Brechkraft der Linse $F_{Linse}$ in die Basisbrechkraft $F_{Basis}$ der zu fertigenden Linse und in die Brechkraft einer Struktur $F_{Struktur}$ wird realisiert, dass die zu fertigenden Linse mit flacheren Radien hergestellt werden kann. Es erfolgen dadurch eine deutliche Reduzierung der Linsendicke, damit des Linsenvolumens und damit auch des Linsengewichts.

Derartige Linsen mit einem erweiterten Fokusbereich finden in optischen Systemen für eine Kamera, ein Mikroskop oder in optischen Messeinrichtungen Anwendung.

Ein Hauptanwendungsgebiet ist eine Intraokularlinse mit variablem Brennweitenbereich. Mit dem spiralförmigen Brechkraftanteil kann ein Fokussierbereich von 0 bis ca. 3,5 dpt bezogen auf eine feste Basisbrechkraft realisiert werden. Eine solche Intraokularlinse wird meist nach Entfernung der natürlichen Linse ins Auge implantiert. Diese kann aber auch zusätzlich zur natürlichen Linse eingesetzt werden.

Die neue Linse wird nach folgenden Schritten hergestellt:

Schritt 1: Berechnung eines zunächst monofokalen, virtuellen Basissystems, welches die Fokussierung für eine feste Dioptrienstellung übernimmt (im Fall einer IOL-Linse beispielsweise 60 dpt für das gesunde menschliche Auge). Das ist der Grundwert der Brechkraft $F_{Linse}$, welche durch die Oberflächenformen der optischen Flächen, der Linsendicke und einer Materialart festgelegt ist.

Schritt 2: Aufteilung des Grundwertes der Brechkraft $F_{Linse}$ in eine Basisbrechkraft $F_{Basis}$ und in die Brechkraft einer Struktur $F_{Struktur}$.
Praktisch hat es sich als günstig erwiesen, mehr als 50% des Grundwertes der Brechkraft $F_{Linse}$ als refraktive Basisbrechkraft $F_{Basis}$ zu realisieren und weniger als 50% des Grundwertes der Brechkraft $F_{Linse}$ als Brechkraft der Struktur $F_{Struktur}$ zu realisieren. Besonders vorteilhaft hinsichtlich des Auftretens von Fehlern ist es mehr als 70% als refraktive Basisbrechkraft $F_{Basis}$ zu realisieren und weniger als 30% als Brechkraft der Struktur $F_{Struktur}$. Die zu fertigenden Linse mit der Basisbrechkraft $F_{Basis}$ entspricht einer herkömmlichen Linse mit zwei optischen Flächen, welche sphärisch und/oder asphärisch und/oder als Freiformfläche ausgebildet werden können. Zumindest eine dieser optischen Flächen dient als Basisfläche für die Realisierung der zusätzlichen Brechkraftverteilung, welche in dem übernächsten Schritt 4 beschrieben wird.

Schritt 3: Bestimmung der Parameter der zusätzlichen spiral- und strukturförmigen Brechkraft $F_{SS}$ durch Addieren der spiralförmigen Brechkraftverteilung $F_{Spirale}$ auf die Brechkraft der Struktur $F_{Struktur}$.

Schritt 4: Aufaddieren oder subtrahieren der in Schritt 3 erhaltenen spiral- und strukturförmigen Brechkraftverteilung Fss(r, phi) = $F_{Spirale}$ + $F_{Struktur}$ auf die optische Wirkung des Basissystems $F_{Basis}$. Im Ergebnis verändert sich die Brechkraft der Linse nichtlinear radienabhängig mit dem Azimutwinkel der Apertur.

Das "Aufaddieren" der spiral- und strukturförmigen Brechkraftverteilung kann durch mehrere Varianten erfolgen, welche jeweils einzeln oder in beliebiger Kombination miteinander eingesetzt werden:

a) "Aufaddieren" eines spiral- und fresnelförmigen Höhenprofils $z_{SF}$(r, phi), welches die spiral- und strukturförmige Brechkraftverteilung Fss aufweist, auf eine der im Schritt 2 berechneten optischen Flächen der Linse mit der Basisbrechkraft $F_{Basis}$. Diese bestimmte optische Fläche ist die nur errechnete Basisfläche mit dem Höhenprofil $z_{Basis}$, zu der das spiral- und fresnelförmige Höhenprofil $z_{SF}$(r, phi) hinzugerechnet wird und so das zu fertigende Profil dieser optischen Fläche festgelegt ist.

b) "Aufaddieren" einer spiral- und strukturförmigen diffraktiven Struktur mit der Zusatzbrechkraft $F_{SSdiffraktiv}$ auf eine der berechneten und gefertigten optischen Flächen der Linse mit der Basisbrechkraft $F_{Basis}$ gemäß Schritt 2.

c) "Aufaddieren" eines spiral- und strukturförmigen Brechungsindexverlaufes $\Delta n_{SS}$ im Material der Linse. In diesem Fall werden die berechneten Flächen gemäß Schritt 2 nicht verändert und so gefertigt.

Schritt 5: Herstellen der ersten optischen Fläche und der zweiten optischen Fläche der Linse mit der Basisbrechkraft $F_{Basis}$ einschließlich dem Aufbringen oder dem Einbringen der spiral- und strukturförmigen Brechkraftverteilung an und/oder auf und /oder innerhalb der Linse.

Herstellungsverfahren für die spiral- und strukturförmige Brechkraftverteilung, sind insbesondere:

aa) Herstellung der optischen Fläche durch Heißprägen oder Spritzgießen
ab) Herstellung der optischen Fläche durch Diamantdrehen
ba) Herstellung durch lithographische Ätzverfahren auf der optischen Fläche
bb) Herstellung durch Diamantdrehen auf der optischen Fläche
ca) Herstellung durch Schleuderguss aus dem flüssigen Zustand
cb) Herstellung durch Ionenimplantation.

Die Varianten a) und/oder b) können auf eine oder auch auf beiden optischen Flächen einer Linse in einer die Wirkung aufteilenden Weise angewendet werden. Diffraktive Optische Elemente können ergänzend oder zusammen mit der Erzeugung der Brechkraftverteilung zur Farbkorrektur eingesetzt werden. Zum Umfang der Erfindung gehören auch andere Verfahren und Maßnahmen, mit denen die erfindungsgemäße spiral- und strukturförmige Brechkraftverteilung in einer Linse erzielt werden kann, beispielsweise durch Einbringen von Nanoteilchen.

Durch das beschriebene Vorgehen erreicht man zum Beispiel bei einer Intraokularlinse eine stetige Variation der zusätzlichen spiralförmigen Brechkraft $F_{Spirale}$ zur Brechkraft des Basissystems zwischen 0 und ca. 3.5 dpt bei einer in vielen Anwendungsfällen hinreichend guten Bildgüte. Durch den Brechkraftanteil der Struktur $F_{Struktur}$ erreicht man eine Verringerung der Linsendicke um bis zu 50%, was zu einer etwa in der gleichen Größenordnung liegenden Volumen- und Gewichtsreduzierung führt.

Die radienabhängige und Azimut-winkelabhängige Brechkraft $F_{Ges}$(r, phi) ergibt sich aus der Summe einer Grundbrechkraft des Basissystems $F_{Basis}$, aus der Brechkraft der zusätzlichen Struktur $F_{Struktur}$ und aus der radien- und winkelabhängigen spiralförmigen Zusatzbrechkraft $F_{Spirale}$(r, phi). Für den rotationssymmetrischen Fall gilt daher:

$$F_{Ges}(r, phi) = F_{Basis}(r) + \left[ F_{Struktur}(r) + F_{Spirale}(r, phi) \right] = \frac{1}{f_{Basis}} + \left[ \frac{1}{f_{Struktur}} + \frac{1}{f_{Spirale}} \right]$$

Da standardisierte Optikverfahren zur Herstellung der Linse mit dem erweiterten Fokusbereich eingesetzt werden, ist diese Linse kostengünstig herstellbar.

Für den Fall a) "Aufaddieren" eines spiral- und fresnelförmigen Höhenprofils auf eine der optischen Flächen der Linse und damit die Realisierung einer spiral- und fresnelförmigen Brechkraftverteilung des Gesamtsystems gelten die nachfolgenden Betrachtungen:

Die Gesamtbrechkraft $F_{Ges}$ setzt sich aus einer Addition der Grundbrechkraft des Basissystems $F_{Basis}$, der Brechkraft einer Fresnelschen Stufenlinse $F_{Fresnel}$ und der spiralförmigen Zusatzbrechkraft $F_{Spirale}$ zusammen.

$F_{Ges}$(r, phi) = $F_{Basis}$ + $F_{Fresnel}$ + $F_{Spirale}$ (r, phi), wobei aus Fertigungsgründen eine Zusammenstellung in den Basisbrechkraftanteil der Linse $F_{Basis}$ und in den spiral- und fresnelförmige Brechkraftanteil $F_{SF}$(r, phi) = $F_{Fresnel}$ +

$F_{Spirale}(r, phi)$ erfolgt.

Da in diesem Fall die Verteilung der Zusatzbrechkraft durch eine Höhenverteilung erreicht wird, gilt

$$z_{Ges}(r, phi) = z_{Basis} + z_{SF}(r, phi)$$

Das Höhenprofil, welches die spiral- und fresnelförmige Zusatzbrechkraft liefert, ist allgemein durch $z_{SF}(r, phi)$ = $z_{Fresnel} + z_{Spirale}(r, phi)$ beschrieben.

Die Grundbrechkraft des Basissystems ergibt sich für sphärische Linsen aus der Formel

$$F_{Basis} = \left[ \frac{n2 - n1}{n1} * \left( \frac{1}{R3} - \frac{1}{R4} \right) + \frac{(n2 - n1)^2 * d}{n1 * n2 * R3 * R4} \right].$$

Dabei ist beispielsweise $R_3$ der Radius der ersten optischen Fläche, welcher real hergestellt wird und $R_4$ ist der Radius der berechneten Basisfläche, auf die die zusätzliche spiral- und fresnelförmige Brechkraft $F_{SF}$ in Form des Höhenprofils $z_{FS}$ "aufaddiert" wird (die additive Höhe $z_{SF}$, welche die Zusatzbrechkraft liefert, kann auch auf den Radius $R_3$ aufaddiert werden oder auf beide Radien $R_3$ und $R_4$ aufgeteilt werden, die Formeln müssen dann entsprechend verändert werden).

Das Höhenprofil $z_{Basis}$ für die errechnete Basisfläche mit dem Radius $R_4$ der sphärischen Linse ergibt sich zu

$$z_{Basis}(x, y) = R_4 - \sqrt{R_4^2 - x^2 - y^2}$$ und mit $r = \sqrt{x^2 + y^2}$ ergeben sich die Daten der Basisfläche in Polarkoordinaten zu

$$z_{Basis}(r) = R_4 - \sqrt{R_4^2 - r^2} .$$

Für den Fall einer sphärischen Basisfläche und einer rotationssymmetrischen Fresnel-Struktur gilt daher

$$z_{Ges}(r, phi) = \left( R_4 - \sqrt{R_4^2 - r^2} \right) + \left[ z_{Fresnel}(r) + z_{Spirale}(r, phi) \right]$$

Die rotationssymmetrische, fresnelförmige Zusatzbrechkraft errechnet sich zu

$$z_{Fresnel}(r) = \sum_{l=2}^{L} e_l * r^l$$

oder zu

$$z_{Fresnel}(r) = \sum_{l=1}^{L} e_l * r^{2*l}$$

Sofern nichtsphärische Basisflächen für die Linse zu Grunde gelegt werden, werden die bekannten Polynome für die Beschreibung nichtsphärischer Flächen zur Bestimmung der optischen Flächen und/oder der Basisfläche verwendet.

Die spiral- und fresnelförmige Zusatzbrechkraft wird durch den additiven Term $z_{SF}(r, phi)$ als Materialhöhe erzeugt, welche auf die optische Basisfläche mit dem Radius $R_4$ aufaddiert oder auch subtrahiert wird. Analoge Betrachtungen gelten auch für asphärische und Freiformflächen, die sich nicht mit einer einfachen Radienangabe beschreiben lassen.

Das spiralförmige Höhenprofil ergibt sich aus $z_{Spirale}(r, phi) = z_{Spirale\,max}(r) * w(phi)$, wobei das Radialpolynom für den maximalen spiralförmigen Höhenanteil als Funktion des Radius $z_{Spirale\,max}(r)$, welches die maximale zu erzielende Dioptrienzahl verkörpert, ist:

$$z_{Spirale\ max}(r) = \sum_{j=2}^{N} c_j * r^j$$

mit r als radiale Höhe und $c_j$ : Koeffizientensatz des Radialpolynoms.

Im einfachsten Fall ist $w(phi) = \dfrac{phi}{2\pi}$ der winkelabhängige, lineare normierte Anteil, mit phi als azimutalem Winkel auf der Basisfläche des Basissystems (Trägerlinse).

Der additive Term $z_{Spirale}(r, phi)$, welcher auf die Basisfläche der Linse addiert wird, ergibt sich aus

$$z_{Spirale}(r, phi) = z_{Spirale\ max}(r) * w(phi) = \sum_{j=2}^{N} c_j * r^j * \frac{phi}{2\pi}$$

Allgemein erhalt man den Höhenanteil an der Brechkraft des Gesamtsystems der Linse zu

$$z_{Ges}(r, phi) = z_{Basis}(r) + \left[ z_{Fresnel}(r) + z_{Spirale}(r, phi) \right]$$

$$z_{Ges}(r, phi) = z_{Basis} + \left[ \sum_{l=2}^{L} e_l * r^l + \sum_{j=2}^{N} c_j * r^j * \frac{phi}{2\pi} \right]$$

Für das Radialpolynom $z_{Spirale\ max}(r)$ kann in analoger Weise auch der Ansatz

$$z_{Spirale\ max}(r) = \sum_{j=1}^{N} c_j * r^{2*j}$$ verwendet werden, ebenso kann

$$z_{Fresnel}(r) = \sum_{l=1}^{L} e_l * r^{2*l}$$

sein.

Im einfachsten Falle reicht es daher bereits aus, die zusätzliche radiale Brechkraftverteilung als Produkt des normierten Azimutwinkels mit der maximal zu erreichenden Dioptrienzahl zu realisieren.

Für den einfachsten Fall des Radialpolynoms $z_{Spirale\ max}(r) = c_1 * r^2$ mit $c_1$ als Koeffizienten vor dem quadratischen Term lautet die Gleichung für den additiven Term also

$$z_{Spirale}(r, phi) = z_{Spirale\ max}(r) * w(phi) = c_1 * r^2 * \frac{phi}{2\pi}$$

Das obige beschriebene Vorgehen stellt einen linearen "Schraubenanstieg" dar.

In dieser Form ist die Abbildungsqualität über den gesamten Dioptrienbereich in etwa gleichbleibend gut.

Oftmals ist es aber gewünscht, bestimmte Dioptrienbereiche wie zum Beispiel die Null-Dioptrien-Position zu bevorzugen. Hierzu ist es notwendig, die lineare Abhängigkeit der z-Höhe vom Winkel zu verlassen.

Der winkelabhängige Anteil kann allgemein durch die Formel

$$w(phi) = \sum_{i=1}^{M} I_i * \exp\left[-a_i * \left(phi - w_i\right)^2\right]$$

beschrieben werden, wobei $w_i$ die Peaklagen (zwischen 0 und $2\pi$),
$I_i$ die Peakintensitäten und $a_i > 0$ die Dämpfungskoeffizienten für die jeweiligen Peaklagen sind.

Beispielsweise lässt sich für M=1; $I_1$=1 und $w_i$=$2\pi$ durch die Funktion

$$z_{Spirale}(r, phi) = z_{Spirale\ max}(r) * w(phi) = \sum_{j=2}^{N} c_j * r^j * \exp\left[-a_1 * \left(phi - 2\pi\right)^2\right]$$

mit $a_1$=0.25 eine Bevorzugung des Null-Dioptrienbereiches realisieren.

Der geringe Anstieg zwischen phi =0 und phi =2 verursacht eine geringe Addition von Brechkraft in diesem Winkelbereich und somit einen größeren Flächenanteil für die Nulldioptrienentfernung.

Im Zusammenhang mit der Optimierung der Linse mit dem erweiterten Fokusbereich lassen sich weitere Vorteile erzielen, indem weitere Freiheitsgrade bei der Dimensionierung zur Verfügung stehen. Beispielsweise erfolgt dies, wenn die radiale Funktion $z_{Spirale\ max}(r)$ ebenfalls einen azimutabhängigen Koeffizientensatz erhält und so das Radialpolynom $z_{Spirale\ max}(r,\ phi)$ bestimmt wird als

$$z_{Spirale\ max}(r, phi) = \sum_{j=2}^{N} c_j(phi) * r^j$$

Daraus ergibt sich der additive Term $z_{Spirale}(r,\ phi)$ allgemein zu

$$z_{Spirale}(r, phi) = z_{Spirale\ max}(r, phi) * w(phi) = \sum_{j=2}^{N} c_j(phi) * r^j * \sum_{i=1}^{M} I_i * \exp\left[-a_i * \left(phi - w_i\right)^2\right]$$

Daher lassen sich für den Winkelterm $w(phi)$ noch weitere Varianten aus der allgemeinen Formel

$$w(phi) = \sum_{i=1}^{M} I_i * \exp\left[-a_i * \left(phi - w_i\right)^2\right]$$

heraus benennen, mit denen man die "Wirkungsdauer" der einzelnen Azimutbereiche steuern kann.

Die bisherigen Ausführungen basierten alle auf einem additiven Term, welcher refraktiv ist und auf eine der optischen Flächen des Basissystems addiert wird.

Der Additionsterm kann natürlich auch in diffraktiver Form vorliegen, d.h. auf die sphärische Trägerfläche des Basissystems wird ein Diffraktives Optisches Element (DOE) aufgebracht, welches eine spiral- und strukturförmige Phasenfunktion besitzt (Fall b)). Die Gestaltung dieser Phasenfunktion erfolgt dabei in vollkommener Analogie zum refraktiven Ansatz. Es eignen sich insbesondere Blaze-Gitter, Sinusgitter und Binär-Gitter.

Die Gitterfrequenz ändert sich radial und winkelabhängig spiralförmig stetig von einem Ausgangswert bis zu einem Maximalwert, welcher der maximalen Brechkraft entspricht.

Die spiralförmige Phasenfunktion ergibt sich zu

$$Phase_{Spirale}(r, phi) = Phase_{max}(r) * w(phi) = \sum_{j=2}^{N} k_j * r^j * w(phi)$$

oder

$$Phase_{Spirale}(r, phi) = Phase_{max}(r) * w(phi) = \sum_{j=1}^{N} k_j * r^{2*j} * w(phi)$$

Die spiralförmige Brechkraft der diffraktiven Struktur ergibt sich zu

$$F_{Spirale\ diffraktiv} = 2k_2 \frac{\lambda}{wl} * w(phi)$$

oder zu $$F_{Spirale\ diffraktiv} = 2k_1 \frac{\lambda}{wl} * w(phi)$$ mit wl als Konstruktionswellenlänge des Diffraktiven Optischen Elements und $\lambda$ als Anwendungswellenlänge.

Der Term $w(phi)$ kann aus den obigen Ausführungen heraus gewählt werden und ist im einfachsten Fall $\frac{phi}{2\pi}$.

Mit $k_1$ als Koeffizient des quadratischen Terms ergibt sich die maximale Brechkraft, $$F_{Spirale\ max\ diffraktiv} = 2k_1 \frac{\lambda}{wl}$$

und der winkelabhängige Term $F_{Spirale\ diffraktiv}(phi)$ ergibt sich zu $$F_{Spirale\ diffraktiv} = 2k_1 \frac{\lambda}{wl} * \frac{phi}{2\pi}$$

In der diffraktiven Ausführung ist die zusätzliche Brechkraft erzeugende Struktur eine Phasenfunktion. Die Phase einer rotationssymmetrischen Struktur ist

$$Phase_{Struktur}(r) = \sum_{l=2}^{L} g_l * r^l$$

oder

$$Phase_{Struktur}(r) = \sum_{l=1}^{L} g_l * r^{2*l}$$

Die Brechkraft der rotationssymmetrischen Struktur in der diffraktiven Ausführung ist

$$F_{Struktur\ diffraktiv} = 2g_2 \frac{\lambda}{wl}$$

oder ist

$$F_{Struktur\ diffraktiv} = 2g_1 \frac{\lambda}{wl}$$

mit wl als Konstruktionswellenlänge des Diffraktiven Optischen Elements und $\lambda$ als Anwendungswellenlänge.

Die Gesamtbrechkraft der Linse ergibt sich im einfachsten Fall für N=1 und L=1 aus einer vergleichsweise starken refraktiven Grundbrechkraft $F_{Basis}$ des monofokalen Basissystems und aus einem relativ kleinen Brechkraftanteil der diffraktiv erzeugten spiralförmigen und rotationssymmetrischen Zusatzbrechkraft $F_{SS\ diffraktiv}$:

$$F_{Ges} = F_{Basis} + \left[ F_{Struktur\ diffraktiv} + F_{Spirale\ diffraktiv} \right] = F_{Basis} + F_{SS\ diffraktiv}$$

$$F_{Ges} = F_{Basis} + \left[ 2g_1 \frac{\lambda}{wl} + 2k_1 \frac{\lambda}{wl} * w(phi) \right]$$

Praktisch wird zuerst die Basisbrechkraft der Linse $F_{Basis}$ hergestellt und die spiral- und strukturförmige Zusatzbrechkraft $F_{SSdiffraktiv}$ auf eine optische Fläche der Basislinse aufgebracht.

So entsteht durch den diffraktiven Anteil ein relativ nur kleiner Farbfehler und die Linse mit dem erweiterten Fokusbereich ist auch für weißes Licht geeignet.

Die spiral- und strukturförmige Zusatzbrechkraft $F_{SSdiffraktiv}$ kann auch auf die beiden optischen Flächen der Linse aufgeteilt werden.

Der spiral- und strukturförmige Additionsterm $F_{SS}$ kann jedoch auch durch die Herstellung eines spiral- und strukturförmigen Brechzahlgradienten $\Delta n_{SS}$ realisiert werden (Fall c)). In DE 10 2009 033 984 A1 ist beispielsweise beschrieben, wie in einem optischen Material inhomogene optische Eigenschaften erzeugbar sind. In Abwandlung des dort beschriebenen Verfahrens lässt sich auch ein spiralförmiger und strukturförmiger Brechungsindex-Verlauf realisieren. Die Eigenschaften und die Gestaltung des Brechzahlgradienten erfolgt dabei in vollkommener Analogie zum refraktiven und zum diffraktiven Ansatz.

Die Gesamtbrechkraft $F_{Ges}$ ergibt sich aus der Basisbrechkraft $F_{Basis}$ des monofokalen Basissystems plus der Zusatzbrechkraft $F_{SS}$, welche durch die spiral- und strukturförmige Brechkrafterhöhung bereitgestellt wird.

Die spiral- und strukturförmige Zusatzbrechkraft Fss (r, phi) ist proportional der Brechungsindexdifferenz $\Delta n_{SS}$(r, phi) gemäß der Formel

$$\Delta n_{SS}(r, phi) = \Delta n_{Struktur} + \Delta n_{Spirale}(r, phi) = \Delta n_{Struktur} + \Delta n_{Spirale\ max}(r, phi) * w(phi).$$

Die Brechungsindexdifferenz $\Delta n_{SS}$(r, phi) läuft stetig von 0 (bei r=0 und phi=0) bis zur maximalen Brechungsindexerhöhung $\Delta n_{Spirale\ max}$ (bei r=D/2 und phi=2$\pi$ wobei die Funktion w(phi) den oben beschriebenen linearen oder allgemeinen Verlauf vorgeben kann.

Dabei wird $\Delta n_{Spirale\ max}$ (r, phi) in Analogie zur Höhe $z_{Spirale\ max}$ oder zur Phasenfunktion Phase$_{Sprirale\ max}$ berechnet und kann gegenüber der Basisbrechzahl $n_2$ der Linse sowohl positiv als auch negativ sein.

Gegenstand der vorliegenden Erfindung sind daher auch beliebige Mischformen aus spiralförmigen und/oder fresnelförmigen Höhenprofil(en), aus spiralförmigen und/oder rotationssymmetrischen Phasenverlauf(en) und/oder aus spiralförmigen und/oder rotationssymmetrischen Brechungsindexverlauf(en), welche den gewünschten zusätzlichen Brechkraftverlauf erzeugen.

Das Höhenprofil und/oder der Phasenverlauf können auf einer der optischen Fläche und/oder auf den zwei optischen Flächen einer Linse verteilt oder kombiniert angeordnet sein.

Die Erfindung wird nachfolgend anhand von Figuren beschrieben. Es zeigen:

| | |
|---|---|
| Figur 1: | Eine "dicke" Linse mit erweitertem Fokusbereich in der Seitenansicht nach einer Lösung, die in DE 10 2011 101 899 A1 beschrieben ist und von der die Erfindung ausgeht |
| Figur 2: | Eine "dünne" Linse mit erweitertem Fokusbereich gemäß der Erfindung in der Seitenansicht |
| Figur 3: | Schematische Darstellung des Vorgehens und der Berechnungsschritte die zur Dimensionierung und Herstellung der "dünnen" Linse führen |
| Figur 4: | Darstellung eines spiralförmigen Brechkraftanteils |
| Figur 5: | Darstellung eines rotationssymmetrischen, gefresnelten Brechkraftanteils |
| Figur 6: | Darstellung des aufaddierten spiralförmigen und rotationssymmetrischen, gefresnelten Brechkraftanteils |
| Figuren 7 bis 10: | Darstellung des aufaddierten spiralförmigen und rotationssymmetrischen, gefresnelten Brechkraftanteils, wobei die Stärke des spiralförmigen Anteils von Figur zu Figur zunimmt |
| Figur 11: | Darstellung einer diffraktiven, spiralförmigen Struktur, welche den spiralförmigen Brechkraftanteil erzeugt |
| Figur 12: | Darstellung einer diffraktiven, rotationssymmetrischen Ringstruktur |
| Figur 13: | Darstellung der aufaddierten diffraktiven, spiralförmigen Struktur und der diffraktiven, rotationssymmetrischen Ringstruktur, welche dem Brechkraftanteil gemäß Figur 6 entspricht |

Figur 14:        Eine Schematische Darstellung einer Intraokularlinse im Auge
Figur 15:        ein Optisches System einer Kamera mit einer "dicken" Linse
Figur 16:        ein Optisches System einer Kamera mit einer "dünnen" Linse mit dem erweiterten Fokusbereich

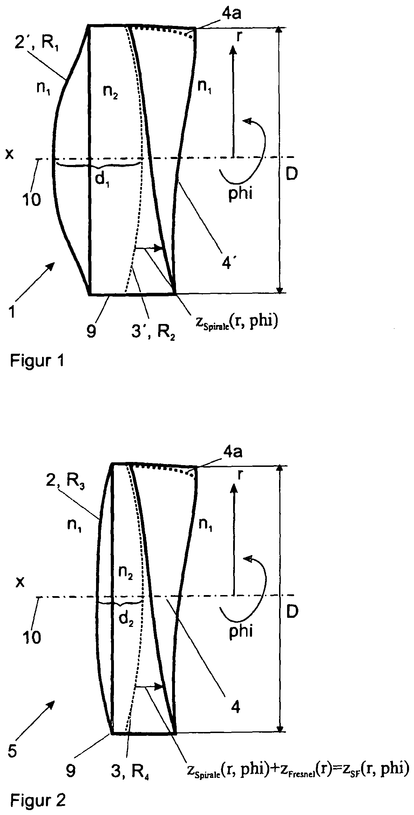

Figur 1 zeigt eine "dicke" Linse 1 mit einem erweiterten Fokusbereich gemäß DE 10 2011 101 899 A1. Gezeigt ist eine Seitenansicht mit Darstellung des spiralförmigen refraktiven Höhenprofils $z_{Spirale}$ (r, phi), welches die spiralförmige Brechkraftverteilung $F_{Spirale}$ (r, phi) erzeugt. Diese Linse 1 ist zunächst durch ihr Basissystem mit dem Radius $R_1$ der ersten optischen Fläche 2' und dem Radius $R_2$ für die errechnete Basisfläche 3', sowie durch die Linsendicke $d_1$ und der Brechungsindex $n_2$ bestimmt. Diese Parameter sind für eine vorgesehene Grundvergrößerung bestimmt. Auf die errechnete Form der Basisfläche 3' mit dem Radius $R_2$ wird eine zusätzliche Materialdicke z "aufaddiert", wobei bei phi=0 die zusätzliche Materialdicke z=0 mm ist, dann stetig ansteigt und bei phi=2 $\pi$ einen maximalen Wert im Millimeterbereich hat. In der Praxis wird der Maximalwert etwas vor dem Azimutwinkel phi=2 $\pi$ liegen, um einen stetigen, wenn auch sehr steilen Übergang zurück zum Wert Null bei phi=0 zu realisieren, wie das durch die mit 4a bezeichnete gestrichelte Kurve angedeutet ist.
Als Beispiel werden Parameter für eine Linse angegeben:

$R_1$= -15,1411 mm herzustellender Radius
$R_2$= 22,3164 mm berechneter Radius
$d_1$= 0,8 mm
$n_1$= 1 (Brechzahl außerhalb der Linse)
$n_2$= 1,56 (Brechzahl des Linsenmediums)

somit ergibt sich aus der Formel

$$f_{Basis} = \cfrac{1}{\left[\cfrac{n2-n1}{n1}*\left(\cfrac{1}{R1}-\cfrac{1}{R2}\right)+\cfrac{(n2-n1)^2*d1}{n1*n2*R1*R2}\right]}$$

die Brennweite der "Basislinse" zu 16,233 mm.
Auf die errechnete Basisfläche mit dem Radius $R_2$= 22,3164 mm wird ein linearer "Schraubenanstieg" gemäß der Formel

$$z_{Spirale}(r, phi) = z_{Spirale\ max}(r)*w(phi) = c_1 * r^2 * \frac{phi}{2\pi}$$

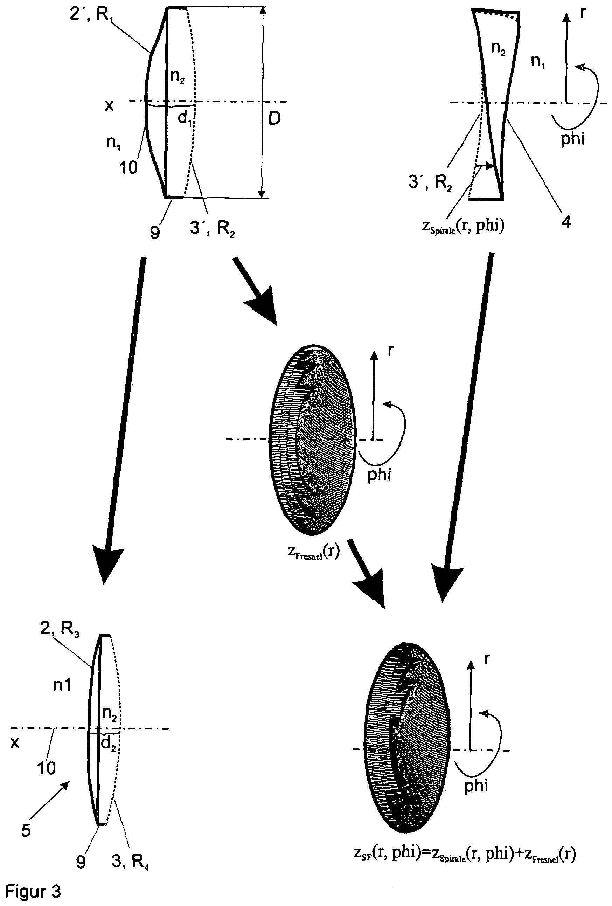

als stetig, linear verlaufendes spiralförmiges Höhenprofil aufaddiert und ergibt die optische Fläche 4'.
Mit $c_1$ =-0,013 erhält man einen spiralförmigen Zusatz, welcher die Brennweite in Luft bis auf 20,57 mm erhöht, was 3,5 dpt entspricht.
Figur 2 zeigt eine erfindungsgemäße "dünne" Linse 5, welche gegenüber der in Figur 1 gezeigten Linse 1, flachere Radien $R_3$ und $R_4$ aufweist und deren Mittendicke $d_2$ dünner ist, wobei annähernd die gleichen Abbildungsparameter der Linse gemäß Figur 1 erzielt werden: Bei einer Grundbrechkraft von etwa 61 dpt wird eine Zusatzbrechkraft von 3,5 dpt erreicht. Die "dünne" Linse 5 weist die Besonderheit auf, dass der spiralförmige Zusatz einen rotationssymmetrischen Fesnel-Anteil enthält. Dieser Fresnel-Anteil realisiert einen Teil der Brechkraft, welche die "dicke" Linse 1 gemäß Figur 1 aus den Radien $R_1$ und $R_2$ sowie der Linsendicke $d_1$ erhält, so dass die Radien $R_3$ und $R_4$ der neuen Linse 5 flacher sind und deren Mittendicke $d_2$ vergleichsweise kleiner ist.
Das Vorgehen zur Dimensionierung der neuen erfindungsgemäßen "dünnen" Linse 5 wird an Hand von Figur 3 erläutert.
Ausgangspunkt ist die in der oberen Zeile der Figur 3 dargestellte "dicke" Linse 1, welche nur berechnet wird. Sie besteht aus dem refraktiven Basissystem mit den Radien $R_1$ und $R_2$ sowie der Mittendicke $d_1$ (links dargestellt) und dem spiralförmigen Brechkraftanteil, welcher theoretisch durch das Höhenprofil $z_{Spirale}$(r, phi) auf dem Radius $R_2$ realisiert wird (rechts dargestellt). Dann ist unterhalb dargestellt, dass die Brechkraft des refraktiven Basissystems aufgeteilt wird, in eine neue "dünne" Linse 5, mit den Radien $R_3$ und $R_4$ sowie mit der Mittendicke $d_2$ und in einen rotationssymmetrischen fresnelförmigen Brechkraftanteil $z_{Fresnel}$(r).
In der unteren Zeile ist dargestellt, dass der fresnelförmige Brechkraftanteil $z_{Fresnel}$(r) und der spiralförmige Anteil $z_{Spirale}$(r, phi) zu einer spiral- und fresnelförmigen Zusatzbrechkraft $F_{SF}$ aufaddiert werden. Auf den nur errechneten

Radius $R_4$ der Basislinse mit dem Höhenprofil $z_{Basis}$ wird das Höhenprofil $z_{SF}(r, phi) = z_{Spirale}(r, phi) + z_{Fresnel}(r)$ aufaddiert.

Hergestellt wird nun eine Linse mit dem Radius $R_3$ mit einer Mittendicke $d_2$ und mit einem Höhenprofil $z_{SF}(r, phi)$ auf dem berechneten Radius $R_4$. Das entsprechende, zu fertigende Höhenprofil der optischen Fläche 4 ergibt sich zu

$$z_{Ges}(r, phi) = \left( R_4 - \sqrt{R_4{}^2 - r^2} \right) + \left[ z_{Fresnel}(r) + z_{Spirale\ max}(r, phi) * w(phi) \right]$$

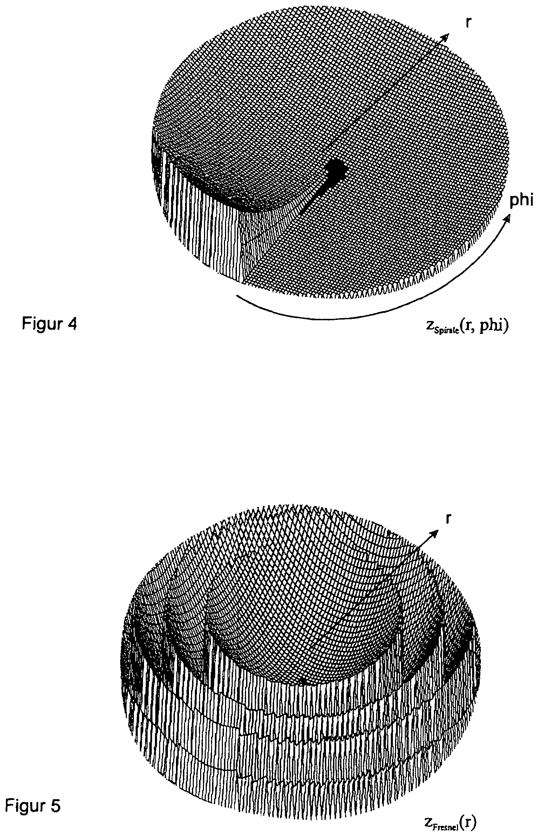

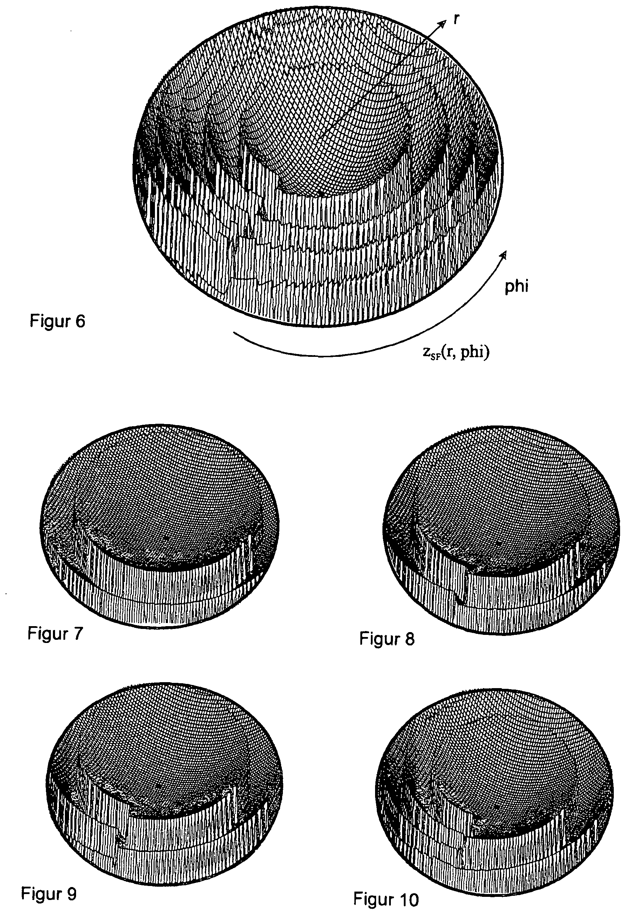

Figur 4 zeigt nur den spiralförmigen Brechkraftanteil $F_{Spirale}$ der Linse als Höhenprofil $z_{Spirale}(r, phi)$. Figur 5 zeigt nur den rotationssymmetrischen, gefresnelten Brechkraftanteil $F_{Fresnel}$ der Linse als Höhenprofil $z_{Fresnel}(r)$. Die Darstellung in Figur 6 zeigt das Ergebnis einer Addition des spiralförmigen Brechkraftanteils und des rotationssymmetrischen, gefresnelten Brechkraftanteils als Höhenprofil $z_{SF}(r, phi)$, welches den spiral- und fresnelförmigen Brechkraftanteil $F_{SF}$ repräsentiert.

Dieses Höhenprofil wird auf das Höhenprofil $z_{Basis}$ der errechneten Basisfläche 3 mit dem Radius $R_4$ aufaddiert und an der herzustellenden Linse gefertigt.

Die Figuren 7 bis 10 zeigen jeweils eine Darstellung des aufaddierten spiralförmigen und rotationssymmetrischen, gefresnelten Brechkraftanteils $F_{SF}$, wobei die Stärke des spiralförmigen Anteils von Figur zu Figur von 1 dpt bis zu 3,5 dpt zunimmt.

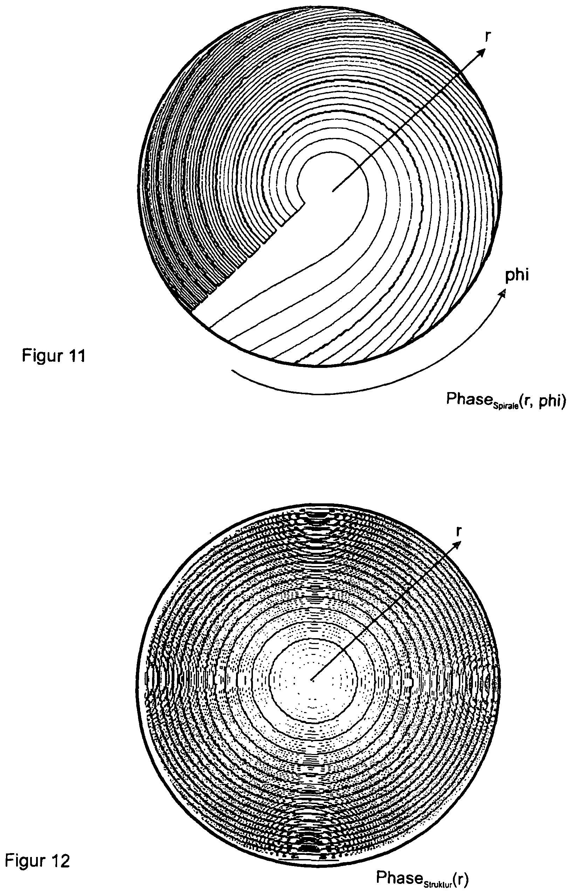

Nachfolgend wird in den Figuren 11 bis 13 gezeigt, dass eine spiral- und strukturförmige Zusatzbrechkraft der Linse Fss ausgehend von dem refraktiven Ansatz (dort ist die strukturförmige Zusatzbrechkraft durch eine Fresnel-Struktur realisiert) in vollständiger Analogie auf einen diffraktiven Ansatz übertragen wird.

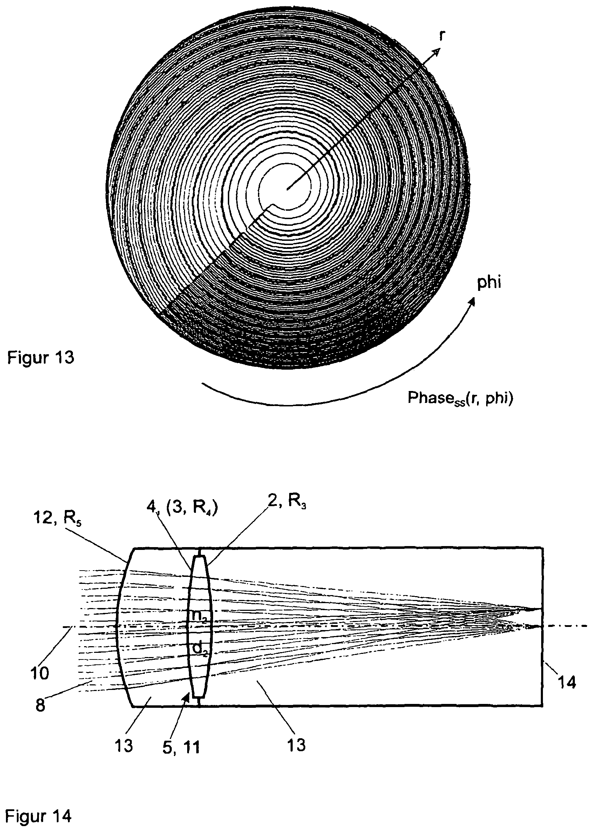

Figur 11 zeigt schematisch eine diffraktive, spiralförmige Struktur, welche den spiralförmigen Brechkraftanteil $F_{Spirale\ diffraktiv}$ erzeugt. Figur 12 zeigt schematisch eine diffraktive, rotationssymmetrische Ringstruktur, welche den strukturförmigen Brechkraftanteil $F_{Struktur\ diffraktiv}$ erzeugt. Figur 13 zeigt das Ergebnis einer Addition der beiden diffraktiven Strukturen. Diese Überlagerung der diffraktiven, spiralförmigen Struktur und der diffraktiven, rotationssymmetrischen Ringstruktur liefert im Ergebnis einen spiral- und strukturförmigen Brechkraftanteil $F_{SS}$, welcher in seiner Wirkung dem refraktiven spiral- und fresnelförmigen Brechkraftanteil $F_{SF}$ entspricht, der durch das spiral- und fresnelförmige Höhenprofil $z_{SF}$ in Figur 6 schematisch dargestellt ist.

Figur 14 zeigt eine schematische Darstellung einer Intraokularlinse 11, welche als "dünne" erweiterte Fokuslinse 5 ins Auge implantiert ist. Im Beispiel ersetzt diese die natürliche Augenlinse und befindet sich im Lichtweg zwischen der Cornea 12 und der Retina 14 im Kammerwasser 13.

Die Intraokularlinse 11 hat eine sphärische erste optische Fläche 2 sowie die Spiral- und Strukturform tragende zweite optische Fläche 4.

In einem ersten Beispiel hat die Intraokularlinse 11 mit dem erweiterten Fokusbereich folgende Parameter für das Basissystem:

Die Basisradien der Trägerlinse sind der hergestellter Linsenradius $R_3$=-20 mm, berechneter Linsenradius $R_4$=+20 mm der Basisfläche 3. Auf die Basisfläche 3 wird das spiral- und fresnelförmige Höhenprofil $z_{SF}(r, phi)$ aufaddiert ist und entsprechend als Fläche 4 gefertigt.

Die Brechkraft des gefresnelten Anteils an der Grundbrechkraft der Linse ergibt sich aus dem Höhenprofil einer Fresnel-Struktur

$$z_{Fresnel}(r) = \sum_{l=1}^{L} e_l * r^{2*l}$$

und ergibt für L=2 die Koeffizienten des rotationssymmetrischen Fresnelpolynomes $e_1$=0,036 und $e_2$=-0,00018398.

Die Brechkraft der spiralförmigen Zusatzbrechkraft der Linse ergibt sich aus dem Höhenprofil

$$z_{Spirale}(r, phi) = z_{Spirale\ max}(r) * w(phi) = \sum_{j=1}^{N} c_j * r^{2*j} * \frac{phi}{2\pi}$$

und ergibt für N=1 den Koeffizienten des Spiralpolynomes $c_1$=0,025.

So ergibt sich

$$z_{SF}(r, phi) = z_{Spirale}(r, phi) + z_{Fresnel}(r) = c_1 * r^2 * \frac{phi}{2\pi} + e_1 * r^2 + e_2 * r^4$$

Die Zackentiefe des gefresnelten Elements p wird mit 0,1 mm gewählt und so ergibt sich die spiralförmige und gefresnelte Zusatzbrechkraft durch die Modulo-Funktion mod($z_{SF}$(r, phi), p).

Dabei sind der Cornea-Radius mit $R_5$=7,814mm angenommen und die Konische Konstante K=-0,26. Der Abstand Cornea 12 bis Linsenvorderseite $R_4$ beträgt 4,12mm, die Linsendicke der Intraokularlinse ist $d_2$ = 0,65 mm und der Abstand von der Linsenvorderseite $R_2$ bis zur Retina 14 beträgt 18,1mm. Als Material wird Benz 25 mit einer Brechzahl des Linsenmediums $n_2$= 1,56 verwendet.

Die Brechzahl außerhalb der Linse, des Kammerwassers 13, ist $n_1$= 1,33.

Im Vergleich dazu hat die in DE 102011 101 899, dort Figur 4, beschriebene Intraokularlinse folgende Parameter:

$R_1$= -15,1411 mm (hergestellte erste optische Fläche 2)
$R_2$= 22,3164 mm (berechnete Basisfläche 3)
Linsendicke $d_1$ = 0,8 mm.

Im Ergebnis ist die neue erfindungsgemäße Linse gemäß dem Beispiel nach Figur 14 um 0,15 mm dünner, was durch den gegenüber $R_1$ flacheren Radius $R_3$ hervorgerufen wird.

In einem zweiten Beispiel zu Figur 14 hat die Intraokularlinse 11 mit dem erweiterten Fokusbereich folgende Parameter für eine diffraktive Ausführung der spiral- und strukturförmigen Zusatzbrechkraft Fss auf einer optischen Fläche der Basislinse:

Cornea : Radius $R_5$ =7,814 mm, asphärische Konuskonstante K=-0,26
Basisradien der Linse $R_3$ =$R_4$ = +/- 20,0 mm (sphärisch), der Abstand Cornea $R_5$ bis zur gefertigten optischen Fläche 4 mit dem Radius $R_4$ ist 4,12 mm,
die Dicke der Intraokularlinse $d_2$ = 0,65 mm und der Abstand der gefertigten optischen Fläche 2 mit dem Radius $R_3$ bis Retina beträgt 18,5 mm.

Als Material für die Intraokularlinse wird "Benz25" eingesetzt. Der Durchmesser der Intraokularlinse beträgt 6 mm. Mit diesen Parametern ist das refraktive Basissystem der Linse beschrieben und seine Brechkraft $F_{Basis}$ ist festgelegt. Die spiralförmige Zusatzbrechkraft $F_{Spirale}$ und die strukturförmige Zusatzbrechkraft $F_{Struktur}$ werden als eine spiral- und strukturförmige diffraktive Brechkraftverteilung $F_{SS}$ mit einem Diffraktiven Optischen Element erzeugt, welches auf die Oberfläche mit dem Radius $R_4$ aufgebracht ist. So ergibt sich die Gesamtbrechkraft der Intraokularlinse 11 zu

$$F_{Ges} == F_{Basis} + F_{SS\ diffraktiv} = F_{Basis} + \left[ F_{Struktur\ diffraktiv} + F_{Spirale\ diffraktiv} \right]$$

Als Phasenfunktion ausgedrückt ergibt sich die spiral- und strukturförmige Zusatzbrechkraft

$$Phase_{SS}(r, phi) = Phase_{Struktur} + Phase_{Spirale}$$

mit

$$Phase_{Strktur}(r) = \sum_{l=1}^{L} g_l * r^{2*l}$$

$$Phase_{Spirale}(r, phi) = Phase_{max}(r) * w(phi) = \sum_{j=1}^{N} k_j * r^{2*j} * w(phi).$$

$$\text{Mit L=2, N=1 und } w(phi) = \frac{phi}{2\pi}$$

$$Phase_{SS}(r, phi) = g_1 * r^2 + g_2 * r^4 + k_1 * r^2 \frac{phi}{2\pi}$$ und die reduzierte Phasenfunktion zu

$$Phase_{SS\ reduziert}(r, phi) = \frac{Phase_{SS}(r, phi)}{wl} - floor\frac{Phase_{SS}(r, phi)}{wl},$$

wobei phi=0 ... $2\pi$ (Azimutwinkel), r = radiale Höhe auf der Linse und wl die Konstruktionswellenlände des diffraktiven Element (synthetische Herstellungswellenlänge) sind.

Für die Koeffizienten des rotationssymmetrischen gefresnelten Anteils, der hier als symmetrischer Anteil eines Diffraktiven Optischen Elementes vorliegt, wird festgelegt: $g_1$=0,006109 und $g_2$=-4,92E-5.

Für den Koeffizient des spiralförmigen Anteils des Diffraktiven optischen Elementes gilt $k_1$=-0,003. Die Profiltiefe der Diffraktiven Optischen Elements ist h = 0,0043 mm.

In einem dritten Beispiel hat die Intraokularlinse 11 mit dem erweiterten Fokusbereich folgende Parameter für eine diffraktive Ausführung der Zusatzbrechkraft Fss, aufgeteilt auf die zwei optischen Flächen 2 und 4 der refraktiven Basislinse 3:

Cornea-Radius $R_5$=7,814 mm, asphärische Konuskonstante K=-0,26)

Die Basisradien der Intraokularlinse sind $R_3$=$R_4$ = +/- 20,0 mm (sphärisch). Weitere Parameter sind: Der Abstand Cornea-Radius $R_5$ bis zur optischen Fläche 4 mit dem Radius $R_4$ von 4,12 mm, die Mittendicke $d_2$ = 0,65 mm und der Abstand der optischen Fläche 2 mit dem Radius $R_3$ bis zur Retina beträgt 18,5 mm, das Linsen-Material ist "Benz25" und der Durchmesser der Intraokularlinse ist 6 mm.

In diesem Beispiel ist auf jeder der optischen Flächen 2 und 4 jeweils ein Diffraktives optisches Element aufgebracht.

Die Brechkraft wird hier so aufgeteilt, dass der spiralförmige Brechkraftanteil $F_{Spirale}$ auf der optischen Fläche mit dem Radius $R_4$ liegt und der strukturförmige rotationssymmetrische Brechkraftanteil $F_{Struktur}$ auf dem Radius $R_3$ liegt.

$$\text{Mit L=2, N=1 und } W(phi) = \frac{phi}{2\pi}$$

ergibt sich

$$Phase_{SS}(r, phi) = g_1 * r^2 + g_2 * r^4 + k_1 * r^2 \frac{phi}{2\pi}$$

und die reduzierte Phasenfunktion zu

$$Phase_{SS\ reduziert}(r, phi) = \frac{Phase_{SS}(r, phi)}{wl} - floor\frac{Phase_{SS}(r, phi)}{wl},$$

wobei phi=0 ... $2\pi$ (Azimutwinkel), r = radiale Höhe auf der Linse und wl die Konstruktionswellenlände des diffraktiven Element (synthetische Herstellungswellenlänge) sind.

Für das auf die optische Fläche 4 aufgebrachte Diffraktive Optische Element gilt:

$g_1$=0, sowie $g_2$=0 und $k_1$=-0,003

Die Profiltiefe der Diffraktiven Optischen Elementes ist h = 0,0043 mm.

Für das auf die optische Fläche 2 aufgebrachte diffraktive optische Element gilt: $g_1$=0,0065 sowie $g_2$=1,8975 E-4 und $k_1$=0.

Die Profiltiefe der Diffraktiven Optischen Elementes ist h = 0,0043 mm.

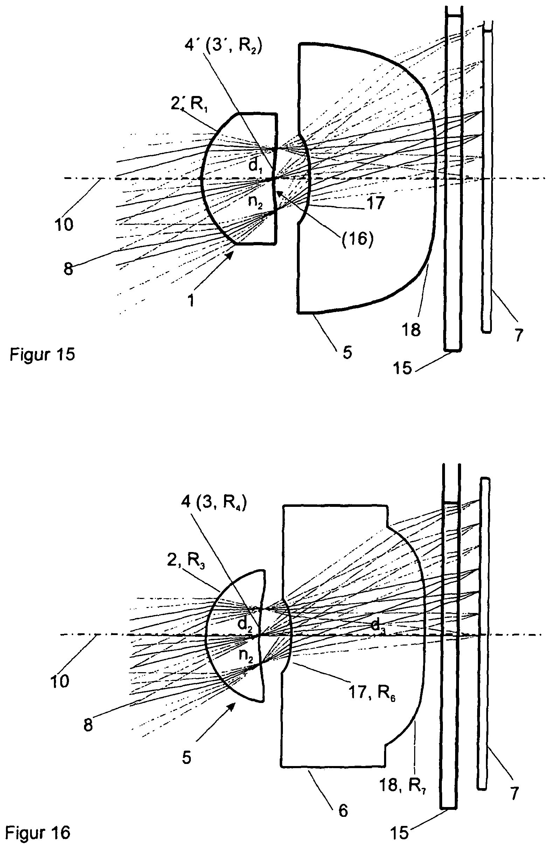

Figur 15 zeigt ein optisches System einer Kamera gemäß Figur 3 aus der DE 10 2011 101 899 A1 mit einer "dicken" Linse 1, welche den erweiterten Fokusbereich aufweist. Zur Basisbrechkraft, resultierend aus den Radien $R_1$, $R_2$, der Brechzahl $n_2$ und der Mittendicke $d_1$ der berechneten Linse wird ein zusätzlicher Brechkraftanteil in Form eines spiralförmigen Brechkraftanteils hinzugerechnet. Dieser spiralförmige Brechkraftanteil wird als Höhenprofil $z_{Spirale}$ auf das Höhenprofil der berechneten Basisfläche 3' hinzu addiert und wird in der Praxis als zweite optische Fläche 4' gefertigt.

Der spiralförmige Brechkraftanteil kann aber auch in diffraktiver Form als Diffraktives Optisches Element auf eine oder beide der optischen Flächen der Linse mit der Basisbrechkraft gefertigt werden. Alternativ kann der spiralförmige Brechkraftanteil auch als Brechungsindexgradient innerhalb der Linse mit der Basisbrechkraft gefertigt werden. Ebenso sind beliebige Mischformen vorgesehen.

Figur 16 zeigt ein optisches System einer Kamera mit einer erfindungsgemäßen "dünnen" Linse 5, welche den erweiterten Fokusbereich aufweist. Im Beispiel wird zur Basisbrechkraft der berechneten Linse ein zusätzlicher Brechkraftanteil in Form eines spiral- und fresnelförmiges Höhenprofils $z_{SF}$ hinzugerechnet. Das Höhenprofil wird auf das Höhenprofil der berechneten Basisfläche 3 mit dem Radius $R_4$ hinzuaddiert und wird als zweite optische Fläche 4 gefertigt. Der spiral- und fresnelförmige Brechkraftanteil kann ganz oder teilweise auch durch ein Diffraktives Optisches Element auf der optischen Fläche der Basislinse (oder verteilt auf beiden optischen Flächen) oder durch einen Brechungsindexgradienten im Linsenmaterial realisiert werden.

In Lichtausbreitungsrichtung folgt der "dünnen" Linse 5 eine asphärische Linse 6 mit den optischen Flächen 17 und 18, dann folgen ein Filter 15 und ein Sensor 7.

Die "dünne" Linse 5 hat objektseitig eine erste optische Fläche 2 mit dem Radius $R_3$. Bildseitig ist die zweite optische Fläche 4 angefertigt, deren Höhenprofil sich aus dem Höhenprofil $z_{Basis}$ des berechneten Radius $R_4$ und dem spiral- und fresnelförmigen Höhenprofil $z_{SF}(r, phi)$ sich ergibt.

Als Beispiel wird ein Handyobjektiv mit einer Brennweite von 5,61 mm gezeigt, welches eine Baulänge von 6,1 mm und eine Öffnung 1:2,8 hat.

Die optischen Flächen 2, 17 und 18 der Linsen 5 und 6 besitzen eine rotationsasphärische Grundform.

Linse 5: Linsendicke $d_2$ = 1,21 mm, Material ist Zeonex

optische Fläche 2: $R_3$ = 1,482 mm konvex

Asphärenkoeffizienten:

K = 0,04649
A = -0,698748E-03
B = 0,987484E-03
C = -0,119379E-03
D = -0,104254E-02
E = 0,323245E-03

Die nicht gefertigte sphärisch konkave Basisfläche 3 hat einen berechneten Radius $R_4$ = 6,303 mm. Auf das Höhenprofil $z_{Basis}$ dieser Basisfläche 3 wird der spiral- und fresnelförmige Brechkraftverlauf in Form eines Höhenprofils $Z_{SF}$ aufaddiert. Dieses Höhenprofil wird dann an der zu fertigenden Linse hergestellt. Im Beispiel sind der Koeffizient des spiralförmigen Polynoms $c_1$ = -0,00268 und der Koeffizient des rotationssymmetrischen Fresnelanteils $e_1$= 0,03

Der zusätzliche spiralförmige Brechkraftanteil berechnet sich zu

$$F_{Spirale} = 2k_1 * r^2 * \frac{phi}{2\pi}$$

und der zusätzliche rotationssymmetrische Fresnel-Anteil zu

$$F_{Fresnel} = 2e_1 * r^2$$

Und die Gesamtbrechkraft ergibt sich zu

$$F_{Ges} = F_{Basis} + F_{SF} = F_{Basis} + F_{Fresnel} + F_{Spirale}$$

$$F_{Ges} = F_{Basis} + 2e_1 * r^2 + 2k_1 * r^2 * \frac{phi}{2\pi}$$

Die auf die Basisfläche 3 aufaddierte Zusatzbrechkraft ist

$$F_{SF} = F_{Fresnel} + F_{Spirale} = 2e_1 * r^2 + 2k_1 * r^2 * \frac{phi}{2\pi}$$

oder als Höhenprofil beschrieben

$$z_{SF} = z_{Fresnel} + z_{Spirale} = 2e_1 * r^2 + 2k_1 * r^2 * \frac{phi}{2\pi}$$

Die Zackentiefe der Fresnelfurchen ist 0,008 mm.

Die Linse 6 hat eine Dicke von 3,0 mm, Material ist Polycarbonat.

Die optische Fläche 17 hat einen Radius $R_6$ = -3,075 mm mit den Asphärenkoeffizienten:

K = 11,058298
A = -0,623991E-01
B = -0,926325E-02
C = 0,244030E-01
D = -0,125809E+00
E = 0,345714E-01
F = -0,101087E-01
G = -0,221418E-15
H = -0,409672E-17
J = 0,991703E-20

Die optische Fläche 18 hat den Radius $R_7$ = 44,1377 mm (konvex) mit den Asphärenkoeffizienten:

K = -0,238656e57
A = -0,171783E-01
B = 0,462293E-03
C = -0,823963E-03
D = 0,227317E-03
E = -0,108925E-04
F = -0,474572E-05
G = 0,385353E-06
H = 0,475909E-07
J = -0,466662E-08

Der Abstand zwischen der Linse 5 bis zur Linse 6 beträgt 0,75 mm, der Abstand zwischen der Linse 6 bis zum Filter 15 beträgt 0,4 mm und der Abstand vom Filter 15 bis zur Bildebene des Detektors 7 beträgt 0,4 mm, wobei die Filterdicke ebenfalls 0,4 mm beträgt.

Das Objektiv liefert einen gleichzeitigen Fokusbereich von 330 mm bis unendlich.

Die Baulänge des Objektives ist nur 6,1 mm und damit um 0,7 mm kleiner als in den Beispielen zu Figur 3 in DE 10 2011 101 899 A1 beschrieben.

Dabei unterstützten insbesondere die zweckmäßige Wahl der Koeffizienten c und e vor dem quadratischen Term die Achromatisierung des Objektivs.

Bezugszeichen

1   "dicke" Linse
2   gefertigte erste optische Fläche (sphärisch, asphärisch, radialsymmetrisch, Freiformfläche) einer "dünnen" Linse
2'   optische Fläche einer "dicken" Linse
3   berechnete Basisfläche der "dünnen" Linse (sphärisch, asphärisch, radialsymmetrisch, Freiformfläche)
3'   berechnete, zweite optische Fläche der "dicken" Linse
4   gefertigte zweite optische Fläche der "dünnen" Linse (sphärisch, asphärisch, radialsymmetrisch, Freiformfläche, spiral- und strukturförmige Oberfläche)
4'   gefertigte Fläche der "dicken" Linse
5   "dünne" Linse
6   asphärische Linse
7   Sensor
8   Lichtbündel
9   Linsenrand
10   optische Achse
11   Intraokularlinse
12   Cornea
13   Kammerwasser
14   Retina
15   Filter
16   spiral- und strukturförmiges Diffraktives Optisches Element (DOE)
17   optische Fläche
18   optische Fläche

| | |
|---|---|
| $F_{Ges}(r, phi)$ | Gesamtbrechkraft der Linse |
| $F_{Linse}\square$ | Grundwert der Brechkraft des Basissystems einer "dicken" Linse |
| $F_{Basis}$ | Brechkraft des Basissystems einer "dünnen" Linse |
| $F_{Struktur}$ | Brechkraft einer Struktur, die zur Brechkraft $F_{Basis}$ der "dünnen" Linse hin zu kommt |
| $F_{Spirale}(r,$ | phi) spiralförmiger Brechkraftanteil, der zur Brechkraft $F_{Basis}$ der "dünnen" Linse hinzu kommt |
| $F_{Spirale\ max}(r, phi)$ | maximale Brechkraft des spiralförmigen Anteils der Brechkraft |
| $F_{SF}(r, phi)$ | spiral- und fresnelförmige zusätzliche refraktive Brechkraft |
| $F_{SS}(r, phi)$ | spiral- und strukturförmige zusätzliche Brechkraft |
| $F_{SS\ diffraktiv}(r, phi)$ | spiral- und strukturförmige zusätzliche diffraktive Brechkraft |
| $F_{Struktur\ diffraktiv}$ | Brechkraft der Struktur in diffraktiver Form |
| $F_{Spirale\ diffraktiv}(r,$ | phi) spiralförmige Brechkraft in diffraktiver Form |
| $f_{Basis}$ | Brennweite des Basissystems |
| $f_{Spirale}(r, phi)$ | Brennweite der spiralförmigen Zusatzbrechkraft |
| $f_{Struktur}$ | Brennweite der Zusatzbrechkraft einer Struktur |
| L, M, N, | Endwerte |
| i, j, l | Zählwerte |
| $c_j, c_1, c_2$ | Polynomkoeffizienten der Spirale für den refraktiven Fall |
| $k_j, k_1, k_2$ | Polynomkoeffizienten der Spirale für den diffraktiven Fall |
| $e_l, e_1, e_2$ | Polynomkoeffizienten des gefresnelten Anteils für den refraktiven Fall |
| $g_l, g_1, g_2$ | Polynomkoeffizienten des strukturförmigen Anteils für den diffraktiven Fall |
| $z_{Spirale\ max}(r)$ | maximale Höhe der Spirale (radiusabhängig) |
| $z_{Spirale\ max}(r, phi)$ | maximale Höhe (radiusabhängig und Azimutwinkel-abhängig) |
| $z_{Spirale}(r, phi)$ | spiralförmige additive Höhe auf der Basisfläche |
| $z_{Basis}$ | Höhenprofil der berechneten Basisfläche 3 |
| $z_{Fresnel}$ | Höhenprofil der Fresnel-Struktur |
| $z_{SF}$ | spiral- und fresnelförmiges Höhenprofilauf der berechneten Basisfläche 3 |
| $z_{Ges}(r, phi)$ | Höhenprofil der gefertigten optischen Fläche 4 |
| w(phi) | winkelabhängiger Anteil des Brechkraftverlaufes |
| $w_i, w_1, w_2$ | Peaklagen der Winkelverteilungsfunktion |
| $a_i, a_1, a_2$ | Dämpfungskoeffizienten für die jeweiligen Peaklagen |
| $l_i, l_1, l_2$ | Intensitätswerte der Einzelpeaks |

| D | Linsendurchmesser |
| $r$ | Radius (radiale Höhe) |
| phi | Azimutwinkel |
| $R_1$ | Radius der ersten optischen Fläche der "dicken" Linse |
| $R_2$ | Radius der optischen Basisfläche der "dicken" Linse |
| $R_3$ | Radius der ersten optischen Fläche 2 der "dünnen" Linse |
| $R_4$ | Radius der optischen Basisfläche 3 der "dünnen" Linse |
| $R_5$ | Cornea-Radius |
| $R_6$ | Radius der asphärischen Linse |
| $R_7$ | Radius der asphärischen Linse |
| $n_1$ | Brechzahl des umgebenden Mediums |
| $n_2$ | Brechzahl des Linsenmaterials |
| $d_1$ | Mittendicke der "dicken" Linse |
| $d_2$ | Mittendicke der "dünnen" Linse |
| $d_3$ | Mittendicke der asphärischen Linse |
| $h$ | Profiltiefe des diffraktiven Elements |
| $p$ | Zackentiefe des gefresnelten Elements |
| $\lambda$ | Anwendungswellenlänge |
| wl | Konstruktionswellenlänge des diffraktiven Elements |
| $\text{Phase}_{\text{Spirale max}}(r, phi)$ | Maximalwert der Gitterfrequenz, welcher der maximalen spiralförmigen Brechkraftverteilung entspricht |
| $\text{Phase}_{\text{Spirale}}(r, phi)$ | Phasenfunktion der spiralförmigen Brechkraftverteilung |
| $\text{Phase}_{\text{Struktur}}$ | Phasenfunktion der zusätzlichen strukturellen Brechkraftverteilung |
| $\text{Phase}_{\text{SS}}(r, phi)$ | Phasenfunktion der spiral- und strukturförmigen Brechkraftverteilung |
| $t$ | Rechengröße |
| floor(t) | ganzzahliger Anteil (Floor-Funktion) |
| Profil(r, phi) | auf die Höhe h reduzierte Phasenfunktion |
| K | asphärische Konstante |
| x, y | kartesische Koordinaten |
| $\Delta n_{\text{Spirale}}$ | spiralförmige Brechungsindexverteilung |
| $\Delta n_{\text{Spirale max}}$ | maximaler Brechungsindex der Spirale |
| $\Delta n_{\text{Struktur}}$ | Brechungsindexverteilung der Struktur |
| $\Delta n_{\text{SS}}$ | spiral- und strukturförmige Brechungsindexverteilung |

## Patentansprüche

1. Linse (5) mit einem erweiterten Fokusbereich, wobei die Linse (5) aus einem transparenten Material besteht sowie eine erste gefertigte optische Fläche (2) und eine zweite gefertigte optische Fläche (4) hat, wobei die Linse (5) eine Brechkraftverteilung $F_{\text{Ges}}$ aufweist, **dadurch gekennzeichnet, dass** sich die Brechkraftverteilung $F_{\text{Ges}}$ der Linse (5) bezogen auf eine zur optischen Achse (10) senkrecht stehende Ebene als Funktion der radialen Höhe (r) und des Azimutwinkels (phi) der Apertur zwischen einem errechneten Grundwert der Brechkraft $F_{\text{Linse}}$ ungleich Null und einem Maximalwert $F_{\text{Spirale max}}(r, phi)$ ändert und somit die Brechkraftverteilung rechnerisch sich zu

$$F_{Ges}(r, phi) = F_{Linse}(r) + F_{Spirale\ max}(r, phi) * w(phi)$$

ergibt, wobei $F_{\text{Spirale max}}(r,phi)$ nichtlinear radienabhängig und ungleich Null ist und w(phi) ein Faktor für den Brechkraftanteil mit dem spiralförmigen Verlauf ist, wobei phi Werte von Null bis $2\pi$ annimmt und w(phi) Werte größergleich Null und kleiner-gleich Eins annimmt, und dass der errechnete Grundwert der Brechkraft $F_{\text{Linse}}$ aufgeteilt ist in einen refraktiven Brechkraftanteil eines Basissystems $F_{\text{Basis}}$ und in einen Brechkraftanteil einer Struktur $F_{\text{Struktur}}$ ungleich Null, wobei die Struktur ein Höhenprofil einer Fresnelschen Stufenlinse oder ein Phasenprofil eines Diffraktiven Optischen Elementes, DOE, oder ein Brechungsindexgradient eines Gradienten-Index-Linse , GRIN-Linse, ist, weiterhin ein spiralförmiger Brechkraftanteil $F_{Spirale}(r, phi) = F_{Spirale\ max}(r, phi) * w(phi)$ und der Brechkraftanteil der Struktur $F_{\text{Struktur}}$ zu einer spiral- und strukturförmigen zusätzlichen Brechkraft $F_{SS}(r, phi) = F_{Struktur} + F_{Spirale}(r, phi)$ zusammengeführt sind, so dass die Gesamtbrechkraft der gefertigten Linse (5) sich zu $F_{Ges}(r,phi) = F_{Basis} + F_{SS}(r,phi)$ ergibt.

2. Linse (5) nach Anspruch 1, **dadurch gekennzeichnet, dass** w(phi) mit der Formel $w(phi) = \dfrac{phi}{2\pi}$ als linearer Verlauf beschrieben ist.

3. Linse (5) nach Anspruch 1, **dadurch gekennzeichnet, dass** die spiral- und strukturförmige Brechkraftverteilung Fss durch ein Höhenprofil erzeugt ist, wobei das Höhenprofil $z_{Ges}$ (r, phi) der zu fertigenden zweiten optischen Fläche (4) aus der Addition eines Höhenprofils $z_{Basis}$ einer berechneten Basisfläche (3), eines fresnelförmigen Höhenprofils $z_{Fresnel}$ einer Fresnelschen Stufenlinse und eines spiralförmigen Höhenprofils $z_{Spirale}$(r, phi) sich ergibt, wobei das additive spiralförmige Höhenprofil $z_{Spirale}$(r, phi) ausgehend von Null bis zu einem Maximalwert $z_{Spirale\ max}$ sich als Funktion $z_{Spirale}$(r, phi) = $z_{Spirale\ max}$(r, phi) * w(phi) ergibt, wobei der Radius (r) zwischen 0 und D/2 und der Azimutwinkel der Apertur (phi) zwischen 0 und 2π sich stetig ändern, wobei das spiralförmige Höhenprofil $z_{Spirale}$(r, phi) und das fresnelförmige Höhenprofil $z_{Fresnel}$ auf das Höhenprofil $z_{Basis}$ der berechneten Basisfläche (3) aufaddiert sind, wodurch die zu fertigende optische Fläche (4) mit einem spiral- und fresnelförmigen Höhenprofil $z_{FS}$ beschrieben ist, wobei gilt

$$z_{Ges}(r, phi) = z_{Basis} + z_{FS}(r, phi) \text{ mit } z_{FS}(r, phi) = z_{Fresnel} + z_{Spirale}(r, phi).$$

4. Linse (5) nach Anspruch 3, **dadurch gekennzeichnet, dass** sich das spiralförmige additive Höhenprofil $z_{Spirale}$ (r, phi) sich ergibt aus dem Produkt eines Polynoms für die maximale Höhe $z_{Spirale\ max}$(r, phi), welches nichtlinear radienabhängig und winkelabhängig ist, und eines winkelabhängigen Anteils w(phi):

$$z_{Spirale}(r, phi) = z_{Spirale\ max}(r, phi) * w(phi),$$

wobei

$$z_{Spirale\ max}(r, phi) = \sum_{j=2}^{N} c_j(phi) * r^j$$

oder

$$z_{Spirale\ max}(r, phi) = \sum_{j=1}^{N} c_j(phi) * r^{2*j}$$

das Polynom für die maximale Höhe ist.

5. Linse (5) nach Anspruch 3, **dadurch gekennzeichnet, dass** sich das additive spiralförmige Höhenprofil $z_{Spirale}$(r, phi) ergibt aus dem Produkt eines Polynoms für die maximale Höhe $z_{Spirale\ max}$(r), welches nichtlinear radienabhängig ist, und einem winkelabhängigen Anteil w(phi):

$$z_{Spirale}(r, phi) = z_{Spirale\ max}(r) * w(phi),$$

wobei

$$z_{Spirale\ max}(r) = \sum_{j=2}^{N} c_j * r^j$$

oder

$$z_{Spirale\ max}(r) = \sum_{j=1}^{N} c_j * r^{2*j}$$

das Radialpolynom für die maximale Höhe ist.

6. Linse (5) nach Anspruch 1, **dadurch gekennzeichnet, dass** die Brechkraftverteilung durch ein Diffraktives Optisches Element erzeugt ist, wobei die berechnete Basisfläche (3) als zweite optische Fläche (4) gefertigt ist und der Brechkraftanteil mit dem spiral- und strukturförmigen Verlauf $F_{SS\ diffraktiv}$ sich aus der Wirkung eines optischen Gitters ergibt, welches auf die gefertigte zweite optische Fläche (4) aufgebracht ist, weiterhin die spiral- und strukturförmige zusätzliche diffraktive Brechkraft $F_{SS\ diffraktiv}$ die Summe aus der Brechkraft der Spirale in diffraktiver Form $F_{Spirale\ diffraktiv}$ und aus der Brechkraft der Struktur in diffraktiver Form $F_{Struktur\ diffraktiv}$ ist und die Brechkraft in Phasenform beschrieben ist als

$$Phase_{SS}(r, phi) = Phase_{Struktur} + Phase_{Spirale}(r, phi),$$

wobei $Phase_{Struktur}$ eine Phasenfunktion der zusätzlichen strukturellen Brechkraftverteilung und $Phase_{Spirale}$ (r, phi) eine Phasenfunktion der spiralförmigen Brechkraftverteilung ist,
wobei der Radius (r) zwischen 0 und D/2 und der Azimutwinkel der Apertur (phi) zwischen 0 und $2\pi$ sich stetig ändern, wodurch das auf der optischen Fläche (3) gefertigte Gitter den spiral- und strukturförmigen Phasenverlauf aufweist.

7. Linse (5) nach Anspruch 6, **dadurch gekennzeichnet, dass** die Werte für das spiralförmige Gitterprofil durch die Formel

$$Phase_{Spirale}(r, phi) = Phase_{Spirale\ max}(r, phi) * w(phi)$$

bestimmt sind, wobei

$$Phase_{Spirale\ max}(r, phi) = \sum_{j=2}^{N} k_j(phi) * r^j$$

oder

$$Phase_{Spirale\ max}(r, phi) = \sum_{j=1}^{N} k_j(phi) * r^{2*j}$$

das Polynom für die maximalen Phasenwerte ist.

8. Linse (5) nach Anspruch 6, **dadurch gekennzeichnet, dass** die Werte für das spiralförmige Gitterprofil durch die Formel

$$Phase_{Spirale}(r, phi) = Phase_{Spirale\ max}(r) * w(phi)$$

bestimmt sind, wobei

$$Phase_{Spirale\ max}(r) = \sum_{j=2}^{N} k_j * r^j \text{ oder } Phase_{Spirale\ max}(r) = \sum_{j=1}^{N} k_j * r^{2*j}$$

das Radialpolynom für die maximalen Phasenwerte ist.

9. Linse (5) nach Anspruch 1, **dadurch gekennzeichnet, dass** der Brechkraftanteil mit dem spiral- und strukturförmigen Verlauf $F_{SS}$ durch eine additive oder subtraktive Brechungsindexverteilung $\Delta n_{SS}$ (r, phi) im Material der Linse sich ergibt, welche die Summe aus einer spiralförmigen Brechungsindexverteilung $\Delta n_{Spirale}$ (r, phi) und aus einer strukturförmigen Brechungsindexverteilung $\Delta n_{Struktur}$ ist, gemäß

$$\Delta n_{SS}(r, phi) = \Delta n_{Struktur} + \Delta n_{Spirale}(r, phi),$$

und die Brechungsindexverteilung von einem Grundwert $n_2$ ausgehend bis zu einem Maximalwert $\Delta n_{Spirale\,max}$ sich als Funktion
$\Delta n_{Spirale}(r, phi) = \Delta n_{Spirale\,max}(r, phi) * w(phi)$ ergibt, wobei der Radius (r) zwischen 0 und D/2 und der Azimutwinkel der Apertur (phi) zwischen 0 und $2\pi$ sich stetig ändern, wodurch für $\Delta n_{SS}$ (r, phi) die spiral- und strukturförmige Brechungsindexverteilung des Linsenmaterials beschrieben ist.

10. Linse (5) nach den Ansprüchen 3, 6 und 9, **dadurch gekennzeichnet, dass** die die zusätzliche spiral- und strukturförmige Brechkraft Fss erzeugenden Formen und/oder Strukturen jeweils einzeln oder miteinander kombiniert auf einer der optischen Flächen (2, 3) der Linse (5) und/oder auch auf den beiden optischen Flächen (2, 3) der Linse (5) einzeln oder in Kombination miteinander und/oder verteilt angeordnet sind und/oder in das Material der Linse (5) eingebracht sind.

11. Linse (5) nach Anspruch 1, **dadurch gekennzeichnet, dass** die Basisbrechkraft der Linse $F_{Basis}$ und/oder die Zusatzbrechkraft der Struktur $F_{Struktur}$ rotationssymmetrisch sind.

12. Verfahren zur Herstellung einer Linse (5) mit einem erweiterten Fokusbereich, welches folgende Schritte umfasst:

Schritt 1: Berechnung eines monofokalen virtuellen Basissystems mit einem Grundwert der Brechkraft $F_{Linse}$ ungleich Null unter Festlegung der Parameter einer ersten optischen Fläche (2'), der Parameter einer zweiten optischen Fläche (3') und einer Linsendicke ($d_1$) sowie einer Werkstoffart mit einem Brechungsindex ($n_2$) und einer Abbe-Zahl,
Schritt 2: Aufteilung des Grundwertes der Brechkraft ($F_{Linse}$) in eine Basisbrechkraft $F_{Basis}$ und in einen strukturförmigen Brechkraftanteil $F_{Struktur}$ ungleich Null, wobei die Basisbrechkraft $F_{Basis}$ durch die Parameter ($R_3$) einer ersten optischen Fläche (2), die Parameter ($R_4$) einer berechneten Fläche (3) und einer Linsendicke ($d_2$) sowie der Werkstoffart mit einem Brechungsindex ($n_2$) und der Abbe-Zahl bestimmt ist, und der strukturförmige Brechkraftanteil $F_{Struktur}$ ein Höhenprofil einer Fresnelschen Stufenlinse oder ein Phasenprofil eines Diffraktiven Optischen Elementes, DOE, oder ein Brechungsindexgradient einer Gradienten-Index-Linse, GRIN-Linse, ist,
Schritt 3: Bestimmung der Parameter einer zusätzlichen spiralförmigen Brechkraftverteilung $F_{Spirale}$(r, phi) ungleich Null, wobei phi Werte von Null bis $2\pi$ annimmt, welche sich bezogen auf eine zur optischen Achse (10) senkrecht stehende Ebene als Funktion der radialen Höhe (r) und des Azimutwinkel (phi) der Apertur zwischen einem Grundwert und einem Maximalwert nichtlinear radienabhängig ändert,
Schritt 4: Bestimmung einer zusätzlichen spiral- und strukturförmigen Brechkraft Fss durch Addieren der spiralförmigen Brechkraftverteilung $F_{Spirale}$ aus Schritt 3 auf die strukturförmige Brechkraftverteilung $F_{Struktur}$ aus Schritt 2,
Schritt 5: Aufaddieren oder Subtrahieren der in Schritt 4 erhaltenen spiral- und strukturförmigen Brechkraftverteilung $F_{SS}$(r, phi) = $F_{Spirale}$ (r, phi)+ $F_{Struktur}$ auf die optische Wirkung des Basissystems $F_{Basis}$ aus Schritt 2,
Schritt 6: Herstellung der Linse (5) mit der spiral- und strukturförmigen Brechkraftverteilung an, auf, und/oder innerhalb der Linse, so dass die Gesamtbrechkraft der gefertigten Linse (5) sich ergibt aus

$$F_{Ges}(r, phi) = F_{Basis} + F_{SS}(r, phi) = F_{Basis} + \left[F_{Struktur} + F_{Spirale}(r, phi)\right].$$

13. Verfahren nach Anspruch 12, bei dem die zusätzliche spiral- und strukturförmige Brechkraft Fss, durch eine Addition eines spiral- und fresnelförmigen Höhenprofils $z_{SF}$(r, phi) auf das Höhenprofil $z_{Basis}$ einer berechneten Basisfläche (3) erzeugt wird, wobei ein spiralförmiges additives Höhenprofil ($z_{Spirale}$) eine Funktion des Radius (r) und des Azimutwinkels der Apertur (phi) ist und sich das spiralförmige additive Höhenprofil ($z_{Spirale}$) sich zwischen dem Wert Null und einem Maximalwert ändert, weiterhin der strukturförmige Brechkraftanteil $F_{Struktur}$ durch ein fresnelförmiges Höhenprofil $z_{Fresnel}$ erzeugt wird und weiterhin die Höhenprofile zu dem spiral- und fresnelförmigen Höhenprofil

($z_{SF}$) addiert werden

$z_{SF}(r, phi) = z_{Fresnel} + z_{Spirale\,max}(r, phi) * w(phi)$ und die Linse (5) mit dem Parameter ($R_3$) für die erste optische Fläche (2) und mit der zweiten optischen Fläche (4) gefertigt wird, wobei das Höhenprofil der zweiten optischen Fläche (4) sich aus $z_{GES}(r, phi) = z_{Basis} + z_{SF}$ ergibt und wobei $z_{Basis}$ das Höhenprofil der errechneten Basisfläche (3) mit dem Radius ($R_4$) ist und wobei $w(phi)$ ein Faktor für den Brechkraftanteil mit einem spiralförmigen Verlauf ist und Werte größer-gleich Null und kleiner-gleich Eins annimmt.

**14.** Verfahren nach Anspruch 12, bei dem sich die Gesamtbrechkraft $F_{Ges}$ der Linse sich ergibt aus $F_{Ges} = F_{Basis} + F_{SS}$, wobei

die spiral- und strukturförmige Brechkraftverteilung $F_{SS}$ durch die Wirkung einer spiral- und strukturförmigen Phasenfunktion Phase $_{SS}$(r, phi) erzeugt wird, welche auf die gefertigte zweite optische Fläche (4) aufgebracht ist, wobei die gefertigte zweite optische Fläche (4) der berechneten Basisfläche (3) mit dem Radius ($R_4$) entspricht und die spiral- und strukturförmige Phasenfunktion bestimmt ist durch $Phase_{SS}(r,phi) = Phase_{Struktur} + Phase_{Spirale}(r,phi)$, wobei die spiralförmige Phasenfunktion $Phase_{Spirale}$ eine Funktion des Radius (r) und des Azimutwinkels der Apertur (phi) ist, und sich die spiralförmige Brechungswirkung zwischen dem Wert Null und einem Maximalwert ändert.

**15.** Verfahren nach Anspruch 12, bei dem die spiral- und strukturförmige Brechkraftverteilung $F_{SS}$ durch einen spiral- und strukturförmigen Brechungsindexverlauf $\Delta n_{SS}$ im Material der Linse hergestellt wird und der spiral- und strukturförmige Brechungsindexverlauf $\Delta n_{SS}$ sich aus der Addition eines spiralförmigen Brechungsindexverlaufes $\Delta n_{Spirale}(r, phi)$ und eines strukturförmigen Brechungsindexverlaufes $\Delta n_{Struktur}$ ergibt, und die spiralförmige Brechkraftverteilung $n_{Spirale}$ sich zwischen dem Grundwert und dem Maximalwert ändert.

**16.** Verfahren nach einem oder mehreren der Ansprüche von 12 bis 15 bei dem eine spiral- und strukturförmige Brechkraftverteilung $F_{SS}$ an und/oder inr eine aphake Intraokularlinse aufgeprägt und/oder eingeprägt wird.

**17.** Objektiv mit einem erweiterten Fokusbereich, **dadurch gekennzeichnet, dass** in einem Strahlengang des Objektivs eine Linse (5) mit einem erweiterten Fokusbereich gemäß der Merkmale in einem oder in mehreren der Ansprüche von 1 bis 11 als abbildendes Element angeordnet ist.

**Claims**

**1.** Lens (5) with an extended range of focus, wherein the lens (5) consists of a transparent material and has a first manufactured optical surface (2) and a second manufactured optical surface (4), wherein the lens (5) has a optical power distribution $F_{Ges}$, **characterized in that**
the optical power distribution $F_{Ges}$ of the lens (5), in relation to a plane perpendicular to the optical axis (10), changes as a function of the radial height (r) and of the azimuth angle (phi) of the aperture between a calculated basic value of the optical power $F_{Linse}$ not equal to zero and a maximum value $F_{Spirale\,max}(r, phi)$, and hence the optical power distribution emerges by calculation as $F_{Ges}(r, phi) = F_{Linse}(r) + F_{Spirale\,max}(r, phi) *w(phi)$, where $F_{Spirale\,max}(r, phi)$ depends nonlinearly on the radius and is unequal to zero and w(phi) is a factor for the optical power component with the spiral profile, wherein phi takes values between 0 and 2n and w(phi) takes values greater than or equal to zero and less than or equal to one,
and **in that** the calculated basic value of the optical power $F_{Linse}$ is split into a refractive optical power component of a base system $F_{Basis}$ and into a optical power component of a structure $F_{Struktur}$ unequal to zero, where the structure is a height profile of a Fresnel lens or a phase profile of a diffractive optical element, DOE, or a refractive index gradient of a gradient index lens, GRIN lens, furthermore a spiral optical power component $F_{Spirale}(r, phi) = F_{Spirale\,max}(r, phi) *w(phi)$ and the optical power component of the structure $F_{Struktur}$ being combined to form a spiral and structure-shaped additional optical power $F_{SS}(r, phi) = F_{Struktur} + F_{Spirale}(r, phi)$ such that the overall optical power of the manufactured lens (5) emerges as

$$F_{Ges}(r, phi) = F_{Basis} + F_{ss}(r, phi).$$

**2.** Lens (5) according to Claim 1, **characterized in that** w(phi) is described as a linear profile using the equation

$$w(phi) = \frac{phi}{2\pi}.$$

3. Lens (5) according to Claim 1, **characterized in that** the spiral and structure-shaped optical power distribution $F_{SS}$ is created by a height profile, wherein the height profile $z_{Ges}$(r, phi) of the second optical surface (4) to be manufactured emerges from adding a height profile $z_{Basis}$ of a calculated base surface (3), a Fresnel-shaped height profile $Z_{Fresnel}$ of a Fresnel lens and a spiral height profile $z_{Spirale}$(r, phi), wherein the additive spiral height profile $z_{Spirale}$(r, phi), starting from zero up to a maximum value $z_{Spirale\ max}$, emerges as a function $z_{Spirale}$ (r, phi) = $z_{Spirale\ max}$(r, phi) *w (phi), wherein the radius (r) changes continuously between 0 and D/2 and the azimuth angle of the aperture (phi) changes continuously between 0 and 2n, wherein the spiral height profile $z_{Spirale}$(r, phi) and the Fresnel-shaped height profile $z_{Fresnel}$ are added to the height profile $z_{Basis}$ of the calculated base surface (3), as result of which the optical surface (4) to be manufactured is described by a spiral and Fresnel-shaped height profile $z_{FS}$, wherein

$$z_{Ges}(r, phi) = z_{Basis} + z_{FS}(r, phi)$$

with

$$z_{FS}(r, phi) = z_{Fresnel} + z_{Spirale}(r, phi)$$

applies.

4. Lens (5) according to Claim 3, **characterized in that** the spiral additive height profile $z_{Spirale}$(r, phi) emerges from the product of a polynomial for the maximum height $z_{Spirale\ max}$(r, phi), which depends nonlinearly on the radius and the angle, and an angle-dependent component w(phi):

$$z_{Spirale}(r, phi) = z_{Spirale\ max}(r, phi) *w(phi),$$

where

$$z_{Spirale\ max}(r, phi) = \sum_{j=2}^{N} c_j(phi) * r^j$$

or

$$z_{Spirale\ max}(r, phi) = \sum_{j=1}^{N} c_j(phi) * r^{2*j}$$

is the polynomial for the maximum height.

5. Lens (5) according to Claim 3, **characterized in that** the additive spiral height profile $z_{Spirale}$ (r, phi) emerges from the product of a polynomial for the maximum height $z_{Spirale\ max}$ (r), which depends nonlinearly on the radius, and an angle-dependent component w(phi):

$$z_{Spirale}(r, phi) = z_{Spirale\ max}(r) *w(phi),$$

where

$$z_{Spirale\ max}(r) = \sum_{j=2}^{N} c_j * r^j$$

or

$$z_{Spirale\,max}(r) = \sum_{j=1}^{N} c_j * r^{2*j}$$

is the radial polynomial for the maximum height.

6. Lens (5) according to Claim 1, **characterized in that** the optical power distribution is produced by a diffractive optical element, wherein the calculated base surface (3) is manufactured as second optical surface (4) and the optical power component with the spiral and structure-shaped profile $F_{SS\,diffraktiv}$ emerges from the effect of an optical grating, which is applied onto the manufactured second optical surface (4), furthermore the spiral and structure-shaped additional diffractive optical power $F_{SS\,diffraktiv}$ being the sum of the optical power of the spiral in diffractive form $F_{Spirale\,diffraktiv}$ and the optical power of the structure in diffractive form $F_{Struktur\,diffraktiv}$ and the optical power in phase form being described as

$$Phase_{SS}(r, phi) = Phase_{Struktur} + Phase_{Spirale}(r, phi),$$

where $Phase_{Struktur}$ is a phase function of the additional structural optical power distribution and $Phase_{Spirale}$ (r, phi) is a phase function of the spiral optical power distribution,
where the radius (r) changes continuously between 0 and D/2 and the azimuth angle of the aperture (phi) changes continuously between 0 and 2n, as a result of which the grating manufactured on the optical surface (3) has the spiral and structure-shaped phase profile.

7. Lens (5) according to Claim 6, **characterized in that** the values for the spiral grating profile are determined by the equation $Phase_{spirale}$ (r, phi) = $Phase_{Spirale\,max}$ (r, phi) *w (phi), where

$$Phase_{Spirale\,max}(r, phi) = \sum_{j=2}^{N} k_j(phi) * r^{j}$$

or

$$Phase_{Spirale\,max}(r, phi) = \sum_{j=1}^{N} k_j(phi) * r^{2*j}$$

is the polynomial for the maximum phase value.

8. Lens (5) according to Claim 6, **characterized in that** the values for the spiral grating profile are determined by the equation

$$Phase_{Spirale}(r, phi) = Phase_{Spirale\,max}(r) *w(phi),$$

where

$$Phase_{Spirale\,max}(r) = \sum_{j=2}^{N} k_j * r^{j}$$

or

$$Phase_{Spirale\ max}(r) = \sum_{j=1}^{N} k_j * r^{2*j}$$

is the radial polynomial for the maximum phase value.

9. Lens (5) according to Claim 1, **characterized in that** the optical power component with the spiral and structure-shaped profile $F_{SS}$ emerges from an additive or subtractive refractive index distribution $\Delta n_{SS}$(r, phi) in the material of the lens, which is the sum of a spiral refractive index distribution $\Delta n_{Spirale}$(r, phi) and a structure-shaped refractive index distribution $\Delta n_{Struktur}$, as per

$$\Delta n_{SS}(r,\ phi)\ =\ \Delta n_{Struktur\ +}\ \Delta n_{Spirale}(r,\ phi)\ ,$$

and the refractive index distribution emerges, proceeding from a basic value $n_2$ up to a maximum value $\Delta n_{Spirale\ max}$, as a function

$$\Delta n_{Spirale}(r,\ phi)\ =\ \Delta n_{Spirale\ max}(r,\ phi)*w(phi),$$

where the radius (r) changes continuously between 0 and D/2 and the azimuth angle of the aperture (phi) changes continuously between 0 and 2n, as a result of which the spiral and structure-shaped refractive index distribution of the lens material is described for $\Delta n_{SS}$(r, phi).

10. Lens (5) according to Claims 3, 6 and 9, **characterized in that**
the forms and/or structures producing the additional spiral and structure-shaped optical power $F_{SS}$ are arranged on one of the optical surfaces (2, 3) of the lens (5), in each case on their own or combined with one another, and/or also arranged individually or combined with one another and/or in a distributed manner on both optical surfaces (2, 3) of the lens (5) and/or introduced into the material of the lens (5) .

11. Lens (5) according to Claim 1, **characterized in that** the base optical power of the lens $F_{Basis}$ and/or the additional optical power of the structure $F_{Struktur}$ is/are rotationally symmetric.

12. Method for producing a lens (5) with an extended range of focus, comprising the following steps:

step 1: calculating a monofocal virtual base system with a basic value of the optical power $F_{Linse}$ unequal to zero while setting the parameters of a first optical surface (2'), the parameters of a second optical surface (3') and a lens thickness ($d_1$) and also a material type with a refractive index ($n_2$) and an Abbe number,
step 2: dividing the basic value of the optical power ($F_{Linse}$) into a base optical power $F_{Basis}$ and into a structure-shaped optical power component $F_{Struktur}$ unequal to zero, wherein the base optical power $F_{Basis}$ is determined by the parameters ($R_3$) of a first optical surface (2), the parameters ($R_4$) of a calculated surface (3) and a lens thickness ($d_2$) and the material type with a refractive index ($n_2$) and the Abbe number, and the structure-shaped optical power component $F_{Struktur}$ is a height profile of a Fresnel lens or a phase profile of a diffractive optical element, DOE, or a refractive index gradient of a gradient index lens, GRIN lens,
step 3: determining the parameters of an additional spiral optical power distribution $F_{Spirale}$ (r, phi) unequal to zero, where phi takes values between 0 and 2n, which optical power distribution, in relation to a plane perpendicular to the optical axis (10), changes as a function of the radial height (r) and of the azimuth angle (phi) of the aperture between a basic value and a maximum value depending nonlinearly on the radius,
step 4: determining an additional spiral and structure-shaped optical power $F_{SS}$ by adding the spiral optical power distribution $F_{Spirale}$ from step 3 to the structure-shaped optical power distribution Fstruktur from step 2,
step 5: adding or subtracting the spiral and structure-shaped optical power distribution $F_{SS}$(r, phi) = $F_{Spirale}$ (r, phi) + $F_{Struktur}$ obtained in step 4 to the optical effect of the base system $F_{Basis}$ from step 2,
step 6: producing the lens (5) with the spiral and structure-shaped optical power distribution at, on and/or inside the lens such that the overall optical power of the manufactured lens (5) emerges as:

$$F_{Ges}(r, phi) = F_{Basis} + F_{SS}(r, phi)$$
$$= F_{Basis} + [F_{Struktur} + F_{Spirale}(r, phi)].$$

**13.** Method according to Claim 12, wherein the additional spiral and structure-shaped optical power $F_{SS}$ is produced by adding a spiral and Fresnel-shaped height profile $z_{SF}(r, phi)$ to the height profile $z_{Basis}$ of a calculated base surface (3), wherein a spiral additive height profile ($z_{Spirale}$) is a function of the radius (r) and of the azimuth angle of the aperture (phi) and the spiral additive height profile ($z_{Spirale}$) changes between the value zero and a maximum value, furthermore the structure-shaped optical power component $F_{Struktur}$ being produced by a Fresnel-shaped height profile $z_{Fresnel}$ and furthermore the height profiles being added to the spiral and Fresnel-shaped height profile ($z_{SF}$)

$$z_{SF}(r, phi) = z_{Fresnel} + z_{Spirale\ max}(r, phi)*w(phi)$$

and the lens (5) with the parameter ($R_3$) for the first optical surface (2) and with the second optical surface (4) being manufactured, wherein the height profile of the second optical surface (4) emerges as

$$z_{Ges}(r, phi) = z_{Basis} + z_{SF}$$

and wherein $z_{Basis}$ is the height profile of the calculated base surface (3) with the radius ($R_4$) and wherein w(phi) is a factor for the optical power component with a spiral profile and takes values greater than or equal to zero and less than or equal to one.

**14.** Method according to Claim 12, wherein the overall optical power $F_{Ges}$ of the lens emerges as

$$F_{Ges} = F_{Basis} + F_{SS},$$

where the spiral and structure-shaped optical power distribution $F_{SS}$ is produced by the effect of a spiral and structure-shaped phase function $Phase_{SS}(r, phi)$, which is applied to the manufactured second optical surface (4), wherein the manufactured second optical surface (4) corresponds to the calculated base surface (3) with the radius ($R_4$) and the spiral and structure-shaped phase function is determined by

$$Phase_{SS}(r, phi) = Phase_{Struktur} + Phase_{Spirale}(r, phi)$$

where the spiral phase function $Phase_{Spirale}$ is a function of the radius (r) and of the azimuth angle of the aperture (phi), and the spiral refractive effect changes between the value zero and a maximum value.

**15.** Method according to Claim 12, wherein the spiral and structure-shaped optical power distribution $F_{SS}$ is produced by a spiral and structure-shaped refractive index profile $\Delta n_{SS}$ in the material of the lens and the spiral and structure-shaped refractive index profile $\Delta n_{SS}$ emerges from adding a spiral refractive index profile $\Delta n_{Spirale}(r, phi)$ and a structure-shaped refractive index profile $\Delta n_{Struktur}$, and the spiral optical power distribution $n_{Spirale}$ changes between the basic value and the maximum value.

**16.** Method according to one or more of Claims 12 to 15, wherein a spiral and structure-shaped optical power distribution $F_{SS}$ is applied onto and/or into an aphakic intraocular lens.

**17.** Lens system with an extended range of focus, **characterized in that** a lens (5) with an extended range of focus in accordance with the features in one or more of Claims 1 to 11 is arranged as an imaging element in a beam path of the lens system.

**Revendications**

**1.** Lentille (5) pourvue d'une zone de mise au point étendue, la lentille (5) comprenant une matière transparente et comportant une première surface optique fabriquée (2) et une deuxième surface optique fabriquée (4), la lentille (5) présentant une distribution de pouvoir réfringent $F_{Ges}$, **caractérisée en ce que** la distribution de pouvoir réfringent $F_{Ges}$ de la lentille (5) par rapport à un plan perpendiculaire à l'axe optique (10) varie en fonction de la hauteur radiale (r) et de l'angle azimutal (phi) de l'ouverture entre une valeur de base calculée du pouvoir réfringent $F_{Linse}$ différente de zéro et une valeur maximale $F_{Spirale\ max}$(r, phi) et donc la distribution de pouvoir réfringent est donnée mathématiquement par

$$F_{Ges}(r,phi) = F_{Linse}(r) + F_{Spirale\ max}(r,phi)*w(phi),$$

$F_{spirale\ max}$(r,phi) dépendant non linéairement du rayon et étant non nul et w(phi) étant un facteur pour la composante de pouvoir réfringent en forme de spirale, phi prenant des valeurs allant de zéro à $2\pi$ et w(phi) prenant des valeurs supérieures ou égales à zéro et inférieures ou égales à un,

et **en ce que** la valeur de base calculée du pouvoir réfringent $F_{Linse}$ est divisée en une composante de pouvoir réfringent réfractif d'un système de base $F_{Basis}$ et une composante de pouvoir réfringent d'une structure $F_{Struktur}$ non égale à zéro, la structure étant un profil de hauteur d'une lentille à échelons de type Fresnel ou un profil de phase d'un élément optique diffractif, DOE, ou un gradient d'indice de réfraction d'une lentille à gradient d'indice, Lentille GRIN,

en outre une composante de pouvoir réfringent en spirale

$F_{spirale}$ (r,phi) = $F_{Spirale\ max}$ (r,phi) *w (phi) et la composante de pouvoir réfringent de la structure $F_{Struktur}$ étant réunie en un pouvoir réfringent supplémentaire en forme de spirale et de structure

$F_{SS}$(r,phi) = $F_{Struktur}$ + $F_{Spirale}$(r,phi) de sorte que le pouvoir réfringent total de la lentille (5) produite est obtenue par

$$F_{Ges}(r, phi) = F_{Basis} + F_{SS}(r,phi).$$

2. Lentille (5) selon la revendication 1, **caractérisée en ce que** w(phi) est décrite comme une courbe linéaire avec la formule $w(phi) = \frac{phi}{2\pi}.$

3. Lentille (5) selon la revendication 1, **caractérisée en ce que** la distribution de pouvoir réfringent en forme de spirale et de structure $F_{SS}$ est générée par un profil de hauteur, le profil de hauteur $z_{Ges}$(r, phi) de la deuxième surface optique (4) à fabriquer étant obtenu par l'addition d'un profil de hauteur $z_{Basis}$ d'une surface de base calculée (3), d'un profil de hauteur de type Fresnel $z_{Fresnel}$ d'une lentille à échelons de type Fresnel et d'un profil de hauteur en forme de spirale $z_{Spirale}$ (r, phi), le profil de hauteur en forme de spirale additif $z_{Spirale}$ (r,phi) étant obtenu de zéro jusqu'à une valeur maximale $z_{Spirale\ max}$ sous la forme d'une fonction $z_{Spirale}$ (r,phi) = $z_{Spirale\ max}$ (r,phi) *w (phi), le rayon (r) variant de manière continue entre 0 et D/2 et l'angle azimutal de l'ouverture (phi) variant de manière continue entre 0 et $2\pi$, le profil de hauteur en forme de spirale $z_{Spirale}$ (r,phi) et le profil de hauteur de type Fresnel $z_{Fresnel}$ étant ajoutés au profil de hauteur $z_{Basis}$ de la surface de base calculée (3) de sorte que la surface optique (4) à fabriquer soit décrite avec un profil de hauteur en forme de spirale et de type Fresnel $z_{FS}$, avec

$$z_{Ges}(r,phi) = z_{Basis} + z_{FS}(r,phi)$$

et

$$z_{FS}(r,phi) = z_{Fresnel} + z_{Spirale}(r,phi)$$

4. Lentille (5) selon la revendication 3, **caractérisée en ce que** le profil de hauteur additif en forme de spirale $z_{Spirale}$ (r, phi) est obtenu par le produit d'un polynôme pour la hauteur maximale $z_{Spirale\ max}$ (r,phi), qui est une fonction non linéaire du rayon et de l'angle, et d'une composante w(phi) :

$$z_{Spirale}(r, phi) = z_{Spirale\ max}(r, phi) * w(phi),$$

où

$$z_{Spirale\,max}(r, phi) = \sum_{j=2}^{N} c_j(phi) * r^j$$

ou

$$z_{Spirale\,max}(r, phi) = \sum_{j=1}^{N} c_j(phi) * r^{2*j}$$

étant le polynôme de la hauteur maximale.

5. Lentille (5) selon la revendication 3, **caractérisée en ce que** le profil de hauteur en forme de spirale additif $z_{Spirale}$ (r,phi) est obtenu par le produit d'un polynôme pour la hauteur maximale $z_{Spirale\,max}$ (r), qui est une fonction non linéaire du rayon et d'un angle, et d'une composante w(phi) :

$$z_{Spirale}(r, phi) = z_{Spirale\,max}(r) * w(phi),$$

où

$$z_{Spirale\,max}(r) = \sum_{j=2}^{N} c_j * r^j$$

ou

$$z_{Spirale\,max}(r) = \sum_{j=1}^{N} c_j * r^{2*j}$$

étant le polynôme radial de la hauteur maximale.

6. Lentille (5) selon la revendication 1, **caractérisée en ce que** la distribution de pouvoir réfringent est générée par un élément optique diffractif, la surface de base calculée (3) étant fabriquée sous la forme d'une deuxième surface optique (4) et la composante de pouvoir réfringent ayant la forme en spirale et la forme de structure $F_{SS\,diffraktiv}$ étant obtenue à partir de l'effet d'un réseau optique qui est appliqué sur la deuxième surface optique (4), en outre le pouvoir réfringent diffractif supplémentaire en forme de spirale et en forme de structure $F_{SS\,diffraktiv}$ étant la somme du pouvoir réfringent de la spirale sous forme diffractive $F_{Spirale\,diffraktiv}$ et du pouvoir réfringent de la structure sous forme diffractive $F_{Struktur\,diffraktiv}$ et le pouvoir réfringent étant décrite sous forme de phase comme *Phase*$_{SS}$ *(r,phi)* = *Phase*$_{Struktur}$ + *Phase*$_{Spirale}$ *(r,phi)*, *Phase*$_{Struktur}$ étant une fonction de phase de la distribution de pouvoir réfringent structurel supplémentaire et *Phase*$_{Spirale}$ *(r,phi)* étant une fonction de phase de la distribution de pouvoir réfringent en forme de spirale,
le rayon (r) variant de manière continue entre 0 et D/2 et l'angle azimutal de l'ouverture (phi) variant de manière continue entre 0 et $2\pi$ de sorte que le réseau fabriqué sur la surface optique (3) présente la courbe de phase en forme de spirale et en forme de structure.

7. Lentille (5) selon la revendication 6, **caractérisée en ce que** les valeurs du profil de réseau en forme de spirale sont déterminées par la formule

$$Phase_{Spirale}(r, phi) = Phase_{Spirale\,max}(r, phi) * w(phi),$$

où

$$Phase_{Spirale\ max}(r, phi) = \sum_{j=2}^{N} k_j(phi) * r^j$$

ou

$$Phase_{Spirale\ max}(r, phi) = \sum_{j=1}^{N} k_j(phi) * r^{2*j}$$

étant le polynôme pour les valeurs de phase maximales.

8. Lentille (5) selon la revendication 6, **caractérisée en ce que** les valeurs du profil de réseau en forme de spirale sont déterminées par la formule

$$Phase_{Spirale}(r, phi) = Phase_{Spirale\ max}(r) * w(phi),$$

où

$$Phase_{Spirale\ max}(r) = \sum_{j=2}^{N} k_j * r^j$$

ou

$$Phase_{Spirale\ max}(r) = \sum_{j=1}^{N} k_j * r^{2*j}$$

étant le polynôme radial pour les valeurs de phase maximales.

9. Lentille (5) selon la revendication 1, **caractérisée en ce que** la composante de pouvoir réfringent ayant la courbe en forme de spirale et en forme de structure $F_{SS}$ est obtenue par une distribution d'indice de réfraction additive ou soustractive $\Delta n_{SS}$ (r, phi) dans la matière de la lentille qui est la somme d'une distribution d'indice de réfraction en forme de spirale $\Delta n_{Spirale}$ (r, phi) et d'une distribution d'indice de réfraction en forme de structure $\Delta n_{Struktur}$, conformément à

$$\Delta n_{SS}(r, phi) = \Delta n_{Struktur} + \Delta n_{Spirale}(r, phi),$$

et la distribution d'indice de réfraction depuis une valeur de base $n_2$ jusqu'à une valeur maximale $\Delta n_{Spirale\ max}$ résulte en la fonction
$\Delta n_{Spirale}$ (r,phi) = $\Delta n_{Spirale\ max}$ (r,phi) *w(phi), le rayon (r) variant de manière continue entre 0 et D/2 et l'angle azimutal de l'ouverture (phi) variant de manière continue entre 0 et $2\pi$, de sorte que la distribution d'indice de réfraction en forme de spirale et en forme de structure de la matière de la lentille soit décrite pour $\Delta n_{SS}$ (r,phi).

10. Lentille (5) selon les revendications 3, 6 et 9, **caractérisée en ce que** les formes et/ou structures qui génèrent le pouvoir réfringent en forme de spirale et en forme de structure supplémentaire $F_{SS}$ sont disposées individuellement ou en combinaison les unes avec les autres sur l'une des surfaces optiques (2, 3) de la lentille (5) et/ou également individuellement ou en combinaison et/ou de manière répartie les unes avec les autres sur les deux surfaces optiques (2, 3) de la lentille (5) et/ou sont incorporées dans la matière de la lentille (5).

11. Lentille (5) selon la revendication 1, **caractérisée en ce que** le pouvoir réfringent de base de la lentille $F_{Basis}$ et/ou le pouvoir réfringent supplémentaire de la structure $F_{Struktur}$ sont à symétrie de révolution.

12. Procédé de fabrication d'une lentille (5) pourvue d'une zone de mise au point étendue, ledit procédé comprenant les étapes suivantes :

étape 1 : calcul d'un système de base virtuelle monofocal avec une valeur de base du pouvoir réfringent $F_{Linse}$ non égale à zéro, avec spécification des paramètres d'une première surface optique (2'), les paramètres d'une deuxième surface optique (3') et une épaisseur de lentille ($d_1$) ainsi qu'un type de matière présentant un indice de réfraction ($n_2$) et un nombre d'Abbe,

étape 2 : division de la valeur de base du pouvoir réfringent ($F_{Linse}$) en un pouvoir réfringent de base $F_{Basis}$ et une composante de pouvoir réfringent en forme de structure $F_{Struktur}$ non égale à zéro, le pouvoir réfringent de base $F_{Basis}$ étant déterminé par les paramètres ($R_3$) d'une première surface optique (2), les paramètres ($R_4$) d'une surface calculée (3) et une épaisseur de lentille ($d_2$) ainsi que le type de matière présentant un indice de réfraction ($n_2$) et le nombre d'Abbe, et la composante de pouvoir réfringent en forme de structure $F_{Struktur}$ étant un profil de hauteur d'une lentille à échelons de type Fresnel ou un profil de phase d'un élément optique diffractif, DOE, ou un gradient d'indice de réfraction d'une lentille à gradient d'indice, Lentille GRIN,

étape 3 : détermination des paramètres d'une distribution de pouvoir réfringent en forme de spirale supplémentaire $F_{Spirale}$ (r, phi) non égale à zéro, phi prenant des valeurs de zéro à $2\pi$ qui varient suivant une relation non linéaire avec le rayon entre une valeur de base et une valeur maximale par rapport à un plan perpendiculaire à l'axe optique (10) en fonction de la hauteur radiale (r) et de l'angle azimutal (phi) de l'ouverture,

étape 4 : détermination d'un pouvoir réfringent en forme de spirale et en forme de structure supplémentaire $F_{SS}$ par addition de la distribution de pouvoir réfringent en forme de spirale $F_{Spirale}$ de l'étape 3 à la distribution de pouvoir réfringent en forme de structure $F_{Struktur}$ de l'étape 2,

étape 5 : addition ou soustraction de la distribution de pouvoir réfringent en forme de spirale et en forme de structure $F_{SS}(r,phi) = F_{Spirale}(r,phi) + F_{Struktur}$ obtenue à l'étape 4 à l'effet optique du système de base $F_{Basis}$ de l'étape 2,

étape 6 : fabrication de la lentille (5) avec la distribution de pouvoir réfringent en forme de spirale et en forme de structure au niveau de, sur et/ou à l'intérieur de la lentille de sorte que le pouvoir réfringent totale de la lentille (5) fabriquée soit obtenue par

$$F_{Ges}(r,phi) = F_{Basis} + F_{SS}(r,phi) = F_{Basis} + [F_{Struktur} + F_{Spirale}(r,phi)].$$

13. Procédé selon la revendication 12, dans lequel le pouvoir réfringent en forme de spirale et en forme de structure supplémentaire $F_{SS}$ est générée par addition d'un profil de hauteur en forme de spirale et de type Fresnel $z_{SF}(r,phi)$ au profil de hauteur $z_{Basis}$ d'une surface de base calculée (3), un profil de hauteur en forme de spirale additif ($z_{Spirale}$) étant fonction du rayon (r) et de l'angle azimutal de l'ouverture (phi) et le profil de hauteur en forme de spirale additif ($z_{Spirale}$) variant entre la valeur zéro et une valeur maximale, en outre la composante de pouvoir réfringent en forme de structure $F_{Struktur}$ étant générée par un profil de hauteur de type Fresnel $z_{Fresnel}$ et en outre les profils de hauteur étant ajoutés au profil de hauteur en forme de spirale et de type Fresnel ($z_{SF}$) $z_{SF}(r,phi) = z_{Fresnel} + z_{Spirale\ max}(r,phi) *w(phi)$ et la lentille (5) étant fabriquée avec le paramètre ($R_3$) pour la première surface optique (2) et la deuxième surface optique (4), le profil de hauteur de la deuxième surface optique (4) étant obtenu à partir de $z_{Ges}(r,phi) = z_{Basis} + z_{SF}$ et $z_{Basis}$ étant le profil de hauteur de la surface de base calculée (3) ayant le rayon ($R_4$) et w(phi) étant supérieur ou égal à zéro et inférieur ou égal à un.

14. Procédé selon la revendication 12, dans lequel le pouvoir réfringent total $F_{Ges}$ de la lentille est obtenue à partir de $F_{Ges} = F_{Basis} + F_{SS}$, la distribution de pouvoir réfringent en forme de spirale et en forme de structure $F_{SS}$ étant générée par l'effet d'une fonction de phase en forme de spirale et en forme de structure $Phase_{SS}(r,phi)$ qui est appliquée sur la deuxième surface optique (4), la deuxième surface optique produite (4) correspondant à la surface de base calculée (3) avec le rayon ($R_4$) et la fonction de phase en forme de spirale et en forme de structure étant déterminée par $Phase_{SS}(r,phi) = Phase_{Structure} + Phase_{Spirale}(r,phi)$, la fonction de phase en forme de spirale $Phase_{Spirale}$ étant une fonction du rayon (r) et de l'angle azimutal de l'ouverture (phi), et l'effet de réfraction en forme de spirale variant entre la valeur zéro et une valeur maximale.

15. Procédé selon la revendication 12, dans lequel la distribution de pouvoir réfringent en forme de spirale et en forme de structure $F_{SS}$ est produite par une courbe d'indice de réfraction en forme de spirale et en forme de structure

$\Delta n_{SS}$ dans la matière de la lentille et la courbe d'indice de réfraction en forme de spirale et en forme de structure $\Delta n_{SS}$ est obtenue par addition d'une courbe d'indice de réfraction en forme de spirale $\Delta n_{Spirale}$ (r,phi) et d'une courbe d'indice de réfraction en forme de structure $\Delta n_{Struktur}$, et la distribution de pouvoir réfringent en forme de spirale $n_{Spirale}$ varie entre la valeur de base et la valeur maximale.

**16.** Procédé selon une ou plusieurs des revendications 12 à 15, dans lequel une distribution de pouvoir réfringent en forme de spirale et en forme de structure $F_{SS}$ est imprimée sur et/ou dans une lentille intraoculaire aphaque.

**17.** Objectif pourvue d'une zone de mise au point étendue, **caractérisée en ce qu'**une lentille (5) pourvue d'une zone de mise au point étendue selon les caractéristiques d'une ou de plusieurs des revendications 1 à 11 est disposée comme élément d'imagerie dans un trajet de faisceau de l'objectif.

Figur 1

Figur 2

$$z_{SF}(r, phi) = z_{Spirale}(r, phi) + z_{Fresnel}(r)$$

Figur 3

Figur 4

$z_{Spirale}(r, phi)$

Figur 5

$z_{Fresnel}(r)$

Figur 6

$z_{SF}(r, phi)$

Figur 7

Figur 8

Figur 9

Figur 10

Figur 11

Phase$_{Spirale}$(r, phi)

Figur 12

Phase$_{Struktur}$(r)

Figur 13

$Phase_{ss}(r, phi)$

Figur 14

4´ (3´, R₂)

2´ R₁

d₁

n₂

17

(16)

10

8

1

18

5

15

7

Figur 15

4 (3, R₄)

2, R₃

d₂

d₃

n₂

8

17, R₆

10

5

6

18, R₇

15

7

Figur 16

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5982543 A
- US 6120148 A
- US 6536899 B1
- US 2006176572 A1
- WO 2010083546 A2

- EP 0622653 A1
- US 2010002310 A1
- DE 102009033984 A1
- DE 102011101899 A1
- DE 102011101899